# EUROPÄISCHE PATENTSCHRIFT

(11) **EP 1 287 361 B1**
(45) Veröffentlichungstag und Bekanntmachung des Hinweises auf die Patenterteilung: **26.09.2007**
(21) Anmeldenummer: 01951358.9
(22) Anmeldetag: 01.06.2001
(51) Int. Cl.: G01N 33/543, G01N 33/553

(54) **OBERFLÄCHE ZUR IMMOBILISIERUNG VON LIGANDEN**
SURFACE FOR THE IMMOBILISATION OF LIGANDS
SURFACE PERMETTANT L'IMMOBILISATION DE LIGANTS

(30) Priorität: 02.06.2000 DE 10027397
(43) Veröffentlichungstag der Anmeldung: 05.03.2003
(73) Patentinhaber: Graffinity Pharmaceuticals Aktiengesellschaft, 69120 Heidelberg (DE)
(72) Erfinder: DICKOPF, Stefan, 69118 Heidelberg (DE); OTTLEBEN, Holger, 69120 Heidelberg (DE); RAU, Harald, 69126 Heidelberg (DE); SEKUL, Renate, 68526 Ladenburg (DE); SCHMIDT, Kristina, 69126 Heidelberg (DE); VETTER, Dirk, 69120 Heidelberg (DE)
(74) Vertreter: Vossius & Partner
(86) Internationale Anmeldenummer: PCT/DE2001/002072
(87) Internationale Veröffentlichungsnummer: WO 2001/092883

(56) Entgegenhaltungen:
- EP-A- 0 485 874
- EP-A- 0 574 000
- EP-A- 0 698 787
- WO-A-00/73796
- WO-A-90/05503
- SPINKE J ET AL: "MOLECULAR RECOGNITION AT SELF-ASSEMBLED MONOLAYERS: OPTIMIZATION OFSURFACE FUNCTIONALIZATION" JOURNAL OF CHEMICAL PHYSICS, NEW YORK, NY, US, Bd. 99, Nr. 9, 1. November 1993 (1993-11-01), Seiten 7012-7019, XP000972391 ISSN: 0021-9606

## Beschreibung

Die vorliegende Erfindung betrifft Bindeoberflächen zur Immobilisierung von Liganden, Ligandenoberflächen, sowie strukturierte Oberflächenarrays die eine Vielzahl gleicher oder unterschiedlicher Liganden präsentieren. Weiter betrifft die Erfindung Verfahren zur Herstellung und die Verwendung solcher Oberflächen, sowie spezifische Bindemoleküle, die zu ihrer Bereitstellung dienen können.

Biomolekulare Interaktionen werden in bekannten Verfahren der Interaktionsanalyse an Rezeptor-Ligandsystemen studiert, wobei der Rezeptor gewöhnlich ein Biomakromolekül (z.B. ein Protein oder eine Einzelstrang-DNA) ist und der Ligand eine "Sonde", ein Molekül meist geringeren Molekulargewichts biologischer oder synthetischer Herkunft darstellt (Peptide, Oligonukleotide oder sog. kleine organische Moleküle). Solche Liganden weisen hochspezifische Strukturmerkmale auf, die bei Vorliegen korrespondierender Strukturen am Rezeptor mit diesem in Wechselwirkung treten können. Die Anbindung an den Rezeptor kann durch einen oder mehrere Liganden erfolgen. Weiterhin werden auch Systeme studiert, bei denen beide Interaktionspartner Makromoleküle darstellen (z.B. Protein-Protein- Wechselwirkungen).

Die Interaktionsanalyse wird in der pharmazeutischen und agrochemischen Industrie zur Wirkstoffsuche eingesetzt. Hierbei gilt es, eine möglichst große Anzahl an verschiedenen Proben in möglichst kurzem Zeitraum zu analysieren. Dabei besteht ein wachsendes Interesse, Testsysteme zu entwickeln, die in der Lage sind, eine große Anzahl an nicht nur identischer sondern auch unterschiedlicher Moleküle gleichzeitig einer bestimmten Testmethode zur Detektion ihres biospezifischen Bindungsverhaltens zu unterziehen, um spezifisch wirkende Moleküle zu identifizieren (High-Throughput Screening, HTS). Mit einer solchen Parallelisierung eng verbunden ist die gleichzeitige Miniaturisierung der Testanordnung und Automatisierung des Testablaufs. Weiterhin findet die Interaktionsanalyse für das Genomstudium (Polymorphismen- (SNP) oder Expressionsmusteranalyse) oder auch in der Lebensmittelanalytik Verwendung.

Es ist von praktischem Vorteil, einen der Interaktions- oder Bindungspartner, d. h. Ligand oder Rezeptor, an eine organische oder anorganische Oberfläche zu binden, wodurch diese aufgrund der Generierung einer spezifischen Grenzschicht Bioaktivität erhält. Die Anbindung erfolgt vorzugsweise kovalent oder durch Adsorption. Die Immobilisierung eines Interaktionspartners erleichtert die Prozeßführung, wie z.B. das Durchführen von Waschschritten, und vermag in Verbindung mit einem geeigneten, meist optischen Detektionsverfahren (z.B. Fluoreszenzmessung) Auskunft über das Vorhandensein und die Stärke der Wechselwirkungen zwischen den Interaktionspartnern auf molekularer Ebene zu geben. Eine wichtige Voraussetzung für die Interpretation solcher Daten, die aus der Wechselwirkung zwischen Rezeptor und Ligand herrühren, ist die Vergleichbarkeit und Reproduzierbarkeit der Ergebnisse.

Bioaktive Oberflächen lassen sich auf verschiedenen organischen oder anorganischen Trägern, wie beispielsweise Cellulose, Silikaten oder Edelmetalloberflächen erzeugen. Zusätzlich können diese Träger gleichzeitig Bestandteile eines Sensorsystems sein. Was die bioaktive Grenzschicht anbelangt, so bieten Filme aus organischen Monolagen (Bain und Whitesides, Angew. Chem. 101 (1989) 522-8; Zhong und Porter, Anal. Chem. (1995) 709A-715A) besondere Vorteile (physikochemische Stabilität, strukturelle Einheitlichkeit), Zur Erzeugung solcher Filme sind Verfahren bekannt, bei denen beispielsweise in einem ersten Schritt Alkylthiole auf Gold chemisorbiert werden. Die langkettigen Moleküle packen sich als hochgeordnete Monoschicht (self assembled monolayer, SAM) auf die Festphase, wobei die Goldatome von den Schwefelfunktionen komplexiert werden. Kurzkettige Alkylthiole, wie Butanthiol bilden jedoch diese hochgeordneten Monoschichten nicht aus (R. Berger, et al., Science 27 (1997), 2021-2024). Solche SAMs, die im oben genannten Fall noch keinerlei spezifische bioaktive Strukturen aufweisen, sind aus der Literatur vorbekannt und mit vielerlei physikalischen Methoden gut charakterisiert worden. In Poirier und Pylant, Science, 272 (1996) 1145-8 finden sich rastertunnelmikroskopische Aufnahmen solcher Monolagen auf Gold. Neben Gold können auch andere Metalle als Festphase für solche Monolagen dienen.

Die Immobilisation eines Interaktionspartners, der der Oberfläche, insbesondere einer Sensoroberfläche, Bioaktivität verleiht, kann über sogenannte Anker erfolgen. Ein geeignetes Ankermolekül umfaßt zumindest zwei funktionelle Einheiten, die an entgegengesetzten Enden des Ankers vorliegen, und die zum einen die Verknüpfung mit der Festphasenoberfläche, zum anderen die Anbindung des zu immobilisierenden Interaktionspartners ermöglichen. Letztere soll nachfolgend als "Kopfgruppe" bezeichnet werden. Das Strukturelement, das diese funktionellen Gruppen verbindet, sollte vorzugsweise so beschaffen sein, daß es zumindest die Ausbildung einer hochgeordneten Monoschicht auf Grundlage der Ankermoleküle erlaubt.

Die Erzeugung einer bioaktiven Oberfläche unter Verwendung solcher Anker kann ein- oder mehrstufig erfolgen. Im Falle des einstufigen Verfahrens wird der Interaktionspartner vor seiner Immobilisierung an die Kopfgruppe des Ankers geknüpft. Geeignete Anker für das Einstufenverfahren sind in DE 199 24 606.8 beschrieben. Hier wird zur Bereitstellung einer Messoberfläche ein vollständiges Ligand-Anker-Konjugat (LAK) auf die Oberfläche aufgebracht. Dabei handelt es sich um ein Molekül, das den zu immobilisierenden Interaktionspartner und die zur Anbindung an die Oberfläche notwendige funktionelle Gruppe mittels einer zur SAM-Bildung befähigten Struktur verbindet.

Mehrstufige Verfahren generieren zunächst eine Bindeoberfläche in Form einer organischen Grenzschicht, die noch nicht die gewünschten spezifischen Strukturmerkmale präsentiert, jedoch zur Anbindung der zu immobilisierenden Interaktionspartner, z.B. der Liganden, geeignet oder dafür aktivierbar ist. Zur Immobilisierung eines Interaktionspartners wird dieser also erst nach Erzeugung einer ersten, nicht bindungsspezifischen Grenzschicht durch kovalente, ionische oder komplexchemische Bindung über die Kopfgruppe der Bindekomponente verknüpft. Hierzu kann die Kopfgruppe direkt oder nach Aktivierung benutzt werden. Eine Auswahl solcher Kopfgruppen ist in EP 485 874 A2 beschrieben.

Ein wesentlicher Vorteil des mehrstufigen Verfahrens liegt darin, dass für jeden zu immobilisierenden Interaktionspartner die gleiche Oberfläche benutzt werden kann. Deshalb ist die daraus resultierende Konzentration der zu immobilisierenden Interaktionspartner auf der Oberfläche ebenfalls gleich, was beim Einstufenverfahren nicht bzw. nicht immer gegeben ist. Dies ist insbesondere für die Vergleichbarkeit und Reproduzierbarkeit der verschiedenen Meßergebnisse von Bedeutung. Zu beachten ist jedoch auch, dass die chemische Homogenität der nicht biospezifischen Bindeoberfläche mit steigender Anzahl gegebenenfalls notwendiger Aktivierungsschritte wieder abnimmt.

Einen schrittweisen Aufbau einer verdünnten Bindeschicht beschreibt WO 98/40739 A1, bei der in einem ersten Schritt Cystamin auf eine Goldoberfläche gebracht wird und die Bereitstellung von Maleimid als Kopfgruppe erst durch eine zweite chemische Reaktion realisiert wird. Nachteilig ist, dass dadurch ein weiterer Aktivierungsschritt erforderlich ist, um eine Bindeoberfläche zu erhalten. Weiterhin verbleiben reaktive Aminofunktionen, die für eine unspezifische Bindung von Makromolekülen - wie Proteinen - verantwortlich sein können.

Bei allen mehrstufigen Verfahren muß gewährleistet sein, dass die Reaktion, mit der der zu immobilisierende Interaktionspartner über die Kopfgruppe an den Anker gebunden wird, selektiv und kontrolliert abläuft. Dabei ist die kovalente Anbindung des zu immobilisierenden Interaktionspartners von Vorteil, weil die dabei generierte bioaktive Oberfläche z.B. bezüglich einsetzbarer Regenerations- oder Lagerungsbedingungen chemisch stabiler ist. Diese Vorteile gelten besonders gegenüber dem nicht-kovalenten Bindesystem Streptavidin/Biotin, das häufig verwendet wird.

Insbesondere bei der Immobilisierung eines kleinen Liganden, etwa eines sogenannten "Kleinen Moleküls", ist die Selektivität und der quantitative Verlauf der Reaktion von größter Bedeutung für die quantitative Auswertung von Bindungsstudien bzw. Interaktionsanalysen. Anders als z.B. bei makromolekularen Erkennungsstrukturen müssen hier oft geringe Unterschiede in der Bindungsstärke dieser Liganden mit dem Rezeptor aufgelöst werden. Dabei kann ein Ligand höherer Affinität, der in geringerer Dichte (Konzentration) als ein anderer, schwächer bindender Ligand angeboten wird, zu einem artifiziell verminderten Signal führen und umgekehrt ein schwächerer Ligand der in höherer Dichte (Konzentration) angeboten wird zu einem artifiziell erhöhten Signal gegenüber einem weiteren Liganden führen.

In der Biochemie werden zur Gewährleistung einer hohen Selektivität und der Möglichkeit der quantitativen Umsetzung beispielsweise Reaktionen thioltragender oder - funktionalisierter Moleküle eingesetzt. Als Reaktionspartner dient dabei häufig ein Makromolekül, das eine Maleimidylgruppe trägt. Bei der Reaktion zwischen dem Thiol und dem Maleimidylrest entsteht als Produkt ein Thiosuccinimid (Michael-Addition) (G.T. Hermanson, Bioconjugate Techniques, Academic Press 1996, 229-252; Suda et al. Biochem. Biophys. Res. Comm. (1999), 261, 276-282).

Aus der Literatur sind eine Reihe von Reak-tionspartnern für die Thiolgruppe bekannt (nachfolgend Mercaptophile genannt), die ebenfalls ihre selektive und quantitative Umsetzung erlauben,. Hierbei sind neben Maleimiden zu nennen:
Iod- und Bromacetamide, Pyridyldithio-Verbindungen (G.T. Hermanson, Bioconjugate Techniques, Academic Press 1996, 229-252; C. Boeckler et al., J. Immun.Meth. (1996) 191, 1-10), Michael-Akzeptoren allgemein, Acrylsäurederivate, wie deren Ester, Amide, Lactone oder Lactame (K. Matsuura et al. J. Mass Spectrom. (1998) 33, 1199-1208; W. Adam et al. J. Org. Chem. (1995) 60, 578-584), Methylen-gem-difluorocyclopropane (T. Taguchi et al., Tetrahedron (1997), 53, 9497-9508), α,β-ungesättigte Aldehyde und Ketone (Chen et al., J. Am. Chem. Soc. (1994) 116, 2661-2662) sowie α,β-ungesättigte Sulfone und Sulfonamide.

Ein wesentlicher Vorteil des Thiol/Mercaptophil-Systems liegt darin, dass unter schonenden Reaktionsbedingungen die Anbindung des zu immobilisierenden Interaktionspartners erfolgen kann (Raumtemperatur, pH-neutral, Pufferlösung verwendbar). Dies ist insbesondere bei instabilen Verbindungen oder Proteinen die leicht denaturieren von Bedeutung. Ein weiterer Vorteil ist darin zu sehen, dass sich die Thiolgruppe als Funktionalität im Vergleich zu Carbonsäure-, Amin- oder Amidresten selten in Wirkstoffen findet. Somit kann eine ungewollte Reaktion zwischen evtl. nach Immobilisierung des einen Interaktionspartners verbleibenden Mercaptophilen und dem Target weitgehend vermieden werden.
Insbesondere beim System Thiol/Maleiimid ist weiter eine hohe Selektivität im Vergleich zu anderen Funktionalitäten, wie Hydroxyl-, Amin-, Carboxyl- oder Hydroxylamingruppen, bekannt. Ebenso zeichnet sich die Bildung der kovalenten Bindung durch eine hohe Reaktionsgeschwindigkeit aus (vgl. Schelté et al., Bioconj. Chem. (2000),11, 118-123).

Obwohl sich solche Reaktionen in der Biokonjugat-Chemie bewährt haben, werden Anker, die sowohl die Thiolgruppe zur Anbindung an die Festphasenoberfläche als auch eine mercaptophile Kopfgruppe besitzen, um einen Interaktionspartner zu immobilisieren, im Stand der Technik nicht offenbart, da sowohl bei der Synthese als auch bei der Generierung der Bindeschicht intra- sowie intermolekulare Nebenreaktionen bis hin zur Polymerisation auftreten können (bezüglich der gezielten Polymerisation von Oligothiolen mit Bis-Maleimiden vgl. L.R. Dix et al., Eur. Polym. J. 31 (1995), 653-658).

In EP 485 874 A2 wird auf dieses Problem hingewiesen, das dadurch umgangen wird, daß ausschließlich Disulfid- und Sulfidgruppen im Anker verwendet werden, um beispielsweise Maleimid als Kopfgruppe zur Immobilisierung von Proteinen (Reaktion mit -SH-Gruppe eines Fab'-Fragments) zu verwenden. Auch EP 698 787 A1 verwendet kurzkettige, Maleimid-tragende Disulfidanker zur Immoblisierung von Proteinen. Anker basierend auf Disulfid- und Sulfidgruppen haben jedoch den Nachteil, daß eine nicht gewünschte räumliche Nachbarschaft der Kopfgruppen erzeugt wird. In diesem Fall können sich die Kopfgruppen gegenseitig bei der Immobilisierungsreaktion behindern.

Um die räumliche Nähe benachbarter Liganden kontrollieren zu können, werden in EP 485 874 A2 und EP 698 787 A1 nicht ausschließlich Ankermoleküle auf die Festphasenoberfläche gebracht. Diese werden vielmehr "verdünnt". Wird nämlich zur Interaktionsanalyse ein Partner, beispielsweise ein Ligand, an einen Festphasenträger gebunden, können sich benachbarte Liganden auf der Oberfläche gegenseitig beziehungsweise die Wechselwirkung des benachbarten Liganden mit dem zu detektierenden Interaktionspartner beeinflussen. Whitesides und Mitarbeiter konnten diesen Effekt anhand der biospezifischen Adsorption von Carboanhydrase an Benzolsulfonamidgruppen, die unter Verwendung von Alkanthiolaten auf Gold immobilisiert wurden, dadurch nachweisen, daß der Grad der unerwünschten irreversiblen Bindung von Carboanhydrase an ein Gemisch aus ligandtragendem Anker (immobilisiertes Sulfonamid) und Verdünnerkomponente (12,15,18-Trioxa-20-hydroxyicosan-1-thiol) mit wachsendem Anteil an Verdünnerkomponente im Gemisch sinkt (J. Am. Chem. Soc. 1995, 117, 12009-10).

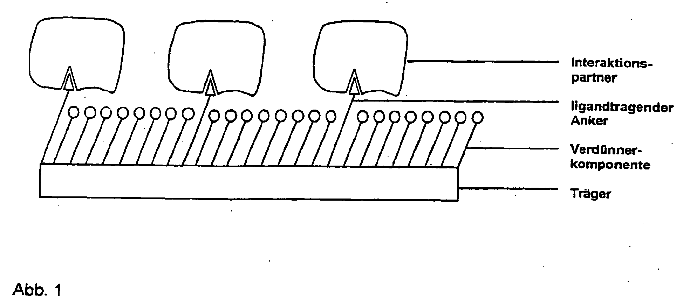

Zur Vermeidung dieses Nachteils können - wie in Abb.1 gezeigt - gemischte Oberflächen aufgebracht werden, die sich aus ligandentragenden Ankermolekülen sowie sogenannten Verdünnermoleküen zusammensetzt, die keine Liganden tragen und so die Meßoberfläche verdünnen.

An die strukturelle Beschaffenheit der Verdünnerkomponente ist die Bedingung geknüpft, daß diese nicht die Interaktion des immobilisierten Interaktionspartners mit dem freien Interaktionspartner beeinflußt. Insbesondere soll möglichst keine spezifische oder unspezifische Bindung zwischen freiem Interaktionspartner und Verdünnerkomponente auftreten (z. B. Verdünner mit möglichst hohe Proteinadsorptionsresistenz). Weiterhin soll zwischen Ankermolekül und Verdünnerkomponente eine möglichst enge strukturelle Änhlichkeit bestehen um zu gewährleisten, dass ihr Mischungsverhalten auf der Festphasenoberfläche möglichst homogen ist

Anker aus dem Stand der Technik, die auf Disulfiden und Sulfiden basieren und damit zwei Kopfgruppen präsentieren, können grundsätzlich keine stochastische Verteilung von kopfgruppentragenden Molekülen und verdünnenden Molekülen gewährleisten. Dies ist jedoch nötig, um später eine gezielte Interaktion der bioaktiven Oberfläche mit dem freien Interaktionspartner sicherzustellen.

Eine Immobilisierung kleiner Liganden (neben Proteinen) in einem Mehrstufenverfahren wird von Lahiri et aL in Anal. Chem. 1999, 71, 777-90, beschrieben. Als Verdünnerkomponente dient 12,15,18-Trioxa-20-hydroxyicosan-1-thiol. Zur Immobilisierung des Liganden trägt das Ankermolekül als Kopfgruppe eine Carbonsäurefunktion, die in einem ersten Schritt mit NHS aktiviert und anschließend mit einer vorhandenen oder eingeführten Aminofunktion im Protein oder Liganden umgesetzt wird. Diese Reaktion ist jedoch nicht vollständig, und zudem ist ihre Ausbeute abhängig von der Verdünnung auf der Oberfläche.

Der vorliegenden Erfindung liegt somit die Aufgabe zugrunde, Bindeoberflächen bereitzustellen, welche reaktive Kopfgruppen präsentieren, die selektive und möglichst quantitative Reaktionen mit zu immobilisierenden Interaktionspartnern eingehen können. Weiterhin befasst sich die Erfindung mit neuartigen Ankermolekülen, die diese Kopfgruppen beinhalten und zusammen mit Verdünnermolekülen zur Bereitstellung solcher Bindeoberflächen eingesetzt werden können. Weiterhin betrifft die Erfindung Sensor bzw. Meßoberflächen, die durch die Immobilisierung spezifischer Interaktionspartnern auf den erfindungsgemäßen Bindeoberflächen erhalten werden können, sowie Array-Anordnungen aus einer Vielzahl gleicher oder unterschiedlicher solcher Meßoberflächen. Schließlich betrifft die Erfindung Verfahren zur Detektion und/oder Quantifizierung einer Wechselwirkung zwischen den oberflächengebundenen und freien Interaktionspartnern, bei denen der freie Interaktionspartner mit einer erfindungsgemäßen Meßoberfläche in Kontakt gebracht wird.
Eine erfindungsgemäße Bindeoberfläche umfaßt einen Festphasenträger, auf dem sich eine organische Monolage spontan organisieren kann, die bevorzugt mindestens zwei Komponenten, eine Binde- und einer Verdünnerkomponente (Anker und Verdünner) umfaßt. Eine entsprechende Anordnung ist in Abb. 1 gezeigt, in der an der Bindekomponente bereits Interaktionspartner immobilisiert sind.

Der Festphasenträger besteht aus einem Substrat, das aus einem Metall, vorzugsweise ein Edelmetall, besonders bevorzugt Gold, gebildet wird bzw. eine Schicht eines solchen Metalles trägt. Die Metallschicht kann gegebenenfalls unter Zuhilfenahme einer Zwischenschicht aufgebracht werden, die zur Haftvermittlung dient Das verwendete Material für das Substrat ist abhängig vom eingesetzten Meßverfahren. Werden reflexionsoptische Verfahren, wie die Oberflächenplwmonenresonanz (SPR) eingesetzt, besteht das Substrat vorteilhafterweise aus Glas oder Kunststoff. Aufgrund der Verwendung schwefelhaltiger Verbindungen zur Immobilisierung von Liganden ist auf solchen Substraten vorzugsweise eine Goldschicht und zur Haftvermittlung eine Chromschicht aufgebracht.

Die räumliche Ausgestaltung des Festphasenträgers zum Einsatz im Rahmen der Erfindung ist nicht eingeschränkt, obwohl insbesondere für Sensorawendungen bevorzugt planare, zweidimensional ausgedehnte Strukturen in Frage kommen. Je nach Anwendungsbereich können aber gegebenenfalls auch dreidimensional gestaltete Formen wie Kugeln oder Hohlkörper verwendet werden. Ein Beispiel für geeignete Festphasenträger sind planare, nicht strukturierte oder physikalisch strukturierte Trägerplatten, die eine Vielzahl separater Felder zur Anbindung der Anker und Verdünner aufweisen.

Verfahren zur Herstellung der Erfindungsgemäßen Bindeschichten gehen von einer Lösung der Ankermoleküle aus, die mit dem Festphasenträger kontaktiert wird. Bevorzugte, verdünnte Meßoberflächen werden erhalten, wenn eine Lösung eingesetzt wird, die eine Mischung von Anker- und Verdünnerkomponente in einem bestimmten molaren Verhältnis enthält Vorzugsweise weist die Lösung ein molares Verhältnis von Anker zu Verdünner auf, das zwischen 1:2 und 1:10.000 liegt Besonders bevorzugte Verdünnungsverhältnisse liegen im Bereich von 1:10 bis 1:100. Die Gesamtkonzentration an Anker- und Verdünnerkomponente liegt vorzugsweise im Bereich von 0,001 bis 100 mmol/l, besonders bevorzugt bei etwa 0,1 bis 1 mmol/l. Zur Erzeugung einer homogenen, großflächigen Bindeoberfläche wird die gesamte Festphasenträgeroberfläche, beispielsweise durch Eintauchen in eine Wanne, mit der (verdünnten) Bindeschicht belegt Zum Aufbringen der Lösung in räumlich definierter Form können handelsübliche Pipettier- oder Tüpfelvorrichtungen, aber auch Mikropipettiervorrichtungen oder Ink-Jet Verfahren verwendet werden. Bevorzugt wird jedoch eine räumliche Strukturierung der Meßoberfläche erst durch gezieltes Aufbringen der zu immobilisierenden Interaktionspartner auf die großflächige Bindeoberfläche erreicht Als geeignete Lösemittel können wäßrige (z.B. Pufferlösungen) oder organische Lösemittel (z.B. Methanol, Ethanol, Acetonitril, N,N-Dimethylformamid, Ethylenglycol) oder Mischungen hiervon verwendet werden.

Während des Aufbringens der Anker auf die Trägeroberfläche können die im Stand der Technik beschriebenen Probleme intramolekularer Reaktionen zwischen den Ankermolekülen dadurch vermieden werden, daß im sauren Medium gearbeitet wird. Zum Einstellen des sauren Millieus enthalten Lösungen der Ankermoleküle vorzugsweise eine Säure, wie Trifluoressigsäure, Salzsäure oder Phosphorsäure.

Ankermoleküle, die zur Bereitstellung der erfindungsgemäßen Bindeoberfläche eingesetzt weden können umfassen zumindest zwei funktionelle Einheiten, die an entgegengesetzten Enden des Ankers vorliegen. Als Gruppierung, die die Verknüpfung des Ankers mit der Oberfläche eines Trägers gewährleistet, kommt in den diesen Ankern eine Thiolgruppe zum Einsatz. Zur Anbindung des zu immobilisierenden Interaktionspartners an den Anker ist dieser mit einer mercaptophilen Kopfgruppe M als zweiter funktioneller Einheit ausgestattet Verbunden sind beide Funktionalitäten mittels eines Strukturelements R, das die Ausbildung einer selbstassemblierenden Monolage, auf der Oberfläche des Trägers ermöglicht Daraus ergibt sich für die im Rahmen der vorliegenden Erfindung eingesetzten Anker die folgende allgemeine Struktur:

Der Rest R besitzt die allgemeine Formel:

-(CH₂)ₐ-Q¹-(CH₂)_{b}-{[Q²-(CH₂)_{c}-[O-(CH₂)_{d}]ₑ-O-(CH₂)_{f}]_{g}-[Q³-(CH₂)_{c}-

[O-(CH₂)_{d'}]_{e'}-O-(CH₂)_{f'}]ₕ}ᵢ-Q⁴-(CH₂)ⱼ-Q⁵-(CH₂)ₖ-

wobei die Variablen, jeweils unabhängig voneinander, wie folgt definiert sind und Zahlenbereiche ihre jeweiligen Grenzen sowie alle darin enthaltenen ganzen Zahlen umfassen sollen:
- Q¹,Q⁵: sind -NH-C(O)-, -C(O)-NH- oder eine Bindung
- Q², Q³, Q⁴: sind -NH-C(O)- oder -C(O)-NH-

- a: ist 5 bis 20, bevorzugt 8 bis 12, besonders bevorzugt 10;
- b: ist0 bis 5, bevorzugt 0 falls Q¹ eine Bindung ist und 1 bis 10, bevorzugt 2 bis 7, besonders bevorzugt 3 bis 5 in allen anderen Fällen;
- c, c': sind 1 bis 5, bevorzugt 1 bis 3, besonders bevorzugt 1;
- d, d': sind 1 bis 5, bevorzugt 1 bis 3, besonders bevorzugt 2;
- e, e': sind 1 bis 5, bevorzugt 1 bis 3, besonders bevorzugt 2;
- f, f': sind 1 bis 5, bevorzugt 1 bis 3, besonders bevorzugt 1;
- g,h: sind 0 bis 3, mit der Maßgabe dass g+h ≥ 1, bevorzugt g+h = 2;
- i: ist 1 bis 3, bevorzugt 1 bis 2, besonders bevorzugt 1;
- j: ist 0 bis 5, bevorzugt 1 bis 3, besonders bevorzugt 2; und
- k: ist 0 bis 5.

Mercaptophile Kopfgruppen M werden ausgewählt aus Iod- und Bromacetamiden Pyridyldithio-Verbindungen, Michael-Akzeptoren, Acrylsäure, (Meth)acrylsäure-estern, -Amiden,-Lactonen oder -Lactamen, Methylen-gem-difluorocyclopropane, α,β-ungesättigte Aldehyden und Ketonen sowie α,β-ungesättigte Sulfonen und Sulfonamiden.

Bevorzugte Kopfgruppen M sind solche der allgemeinen Formel 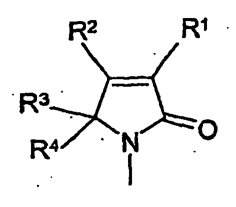 wobei
R¹ und R² unabhängig voneinander Wasserstoff oder C₁ bis C₅ Alkyl, bevorzugt Methyl, Ethyl oder n-Propyl, sind,
R³ und R⁴ unabhängig voneinander Wasserstoff oder C₁ bis C₅ Alkyl, bevorzugt Methyl, Ethyl oder n-Propyl, oder R³ und R⁴ zusammen =O sind und
die Anbindung an den restlichen Anker über den Stickstoff erfolgt.

Bevorzugt sind R³ und R⁴ zusammen =O, am meisten bevorzugt ist die Kopfgruppe ein Maleimidylrest.

Verfahren zur Synthese eines Ankermoleküls können an fester Phase oder in Lösung durchgeführt werden. Bei der Darstellung in Lösung wird bevorzugt die Thiolgruppe mit eine Schutzgruppe versehen Geeignete Schutzgruppen sind in T.W. Greene, P.G.M. Wuts, Protective Groups in Organic Synthesis, Wiley 1999, Kapitel *6 Protection for the Thiol Group,* beschrieben. Bei der Festphasensynthese findet die Ankopplung an das Harz bevorzugt über die Thiolgruppe statt, wodurch diese während der Synthese maskiert bleibt Die Abspaltung von der festen Phase bzw. der Schutzgruppe findet in saurem Milieu (beispielsweise 1 % TFA in Dichlormethan) statt. Es wurde nämlich festgestellt, daß auf diese Weise die in EP 485 874 A2 beschriebenen Nebenreaktionen des Thiols mit dem Mercaptophil vermieden werden können.

Die Verdünnerkomponente umfaßt Verdünnermolekülen der allgemeinen Formel

HS-R-X

in der R wie vorstehend definiert ist und X eine nicht mercaptophile Kopfgruppe darstellt.

Idealerweise dürfen Verdünnermoleküle nicht die Interaktion des immobilisierten Interaktionspartners mit dem freien Interaktionspartner beeinflussen. Insbesondere soll möglichst keine spezifische oder unspezifische. Bindung zwischen freiem Interaktionspartner und Verdünnermolekül auftreten. Weiterhin soll zwischen Ankermolekül und Verdünnermolekül eine möglichst enge strukturelle Änhlichkeit bestehen, um zu gewährleisten, daß das chemische Verhalten auf der Festphasenoberfläche ebenfalls ähnlich ist (homogene Mischbarkeit auf der Festphasenoberfläche). Vorteilhafterweise ist die Gesamtkettenlänge des Verdünnermoleküls im Vergleich zum Ankermolekül verkürzt, beispielsweise um eine Struktureinheit, wie um eine Ethylenglycoleinheit. Dies kann z.B. dadurch erreicht werden, dass bei einem linearen Aufbau des Ankers und des Verdünners aus kommerziell erhältlichen Synthesebausteinen der letzte Baustein bei der Verdünnerstruktur weggelassen wird.

Insbesondere soll die Kopfgrüppe des Verdünnermoleküls verschieden zu der des Ankermoleküls sein. Bevorzugte Kopfgruppen für das Verdünnermolekül sind Methoxy- und Acylamidgruppen. Besonders bevorzugt ist, insbesondere bei Verwendung von Maleimidyl als Ankerkopfgruppe, Acetamid.

Eine erfindungsgemäße (verdünnte) Meßoberfläche wird durch chemische Umsetzung des zu immobilisierenden Interaktionspartners mit der mercaptophilen Kopfgruppe M des Ankers erzeugt. Hierzu trägt der zu immobilisierende Interaktionspartner eine Thiolgruppe, die durch Ausbildung einer kovalenten Bindung mit der Kopfgruppe des Ankers möglichst selektiv und quantitativ reagiert (vgl. C. Boeckler et al., J. Immun. Meth. (1996) 191, 1-10). Ist keine geeignete Funktionalität vorhanden, kann diese durch Umwandlung vorhandener funktioneller Gruppen im zu immobilisierenden Interaktionspartners erzeugt werden, wie z.B. durch reduktive Spaltung einer Disulfidbindung zu einem Thiol (P. Parham, J. Immunol. 131 (1983), 2895-2902). Weiterhin besteht die Möglichkeit, den zu immobilisierenden Interaktionspartner durch chemische Reaktion mit einem Zusatzmolekül, wie beispielsweise Cystein, zu versehen, das die geeignete funktionelle Gruppe zur Anbindung an die Kopfgruppe des Ankers trägt. (Zur Einführung eines geschützten 2-Aminoethylthiols an Oligonukleotide vgl. D. Gottschling et al., Bioconjugate Chem. 1998 (9) 831-837.)

Das Zusatzmolekül kann zusätzlich die Funktion eines Spacers zwischen der Kopfgruppe des Ankers und dem immobilisierten Interaktionspartner besitzen, wodurch letzterer von der Kopfgruppe entfernt gehalten wird und daher deren elektronische und sterische Eigenschaften nicht die Bindungseigenschaften des Interaktionspartners beeinflussen.

Im Rahmen der vorliegenden Erfindung sind vereinfachend unter zu immobilisierenden/immobilisierten Interaktionspartnern oder Liganden chemisch unmodifizierte, oder modifizierte, d.h. mit Thiolfunktionalität versehene Interaktionspartner oder Liganden zu verstehen.

Weiter werden im Kontext der vorliegenden Erfindung als immobilisierte Interaktionspartner allgemein Strukturelemente bezeichnet, die aufgrund ihrer strukturellen Eigenarten spezifische Wechselwirkungen mit Testsubstanzen oder deren Untereinheiten eingehen können. Mit Hilfe solcher immobilisierter Strukturen können, z.B. im Rahmen von Screening-Verfahren, Targets gebunden werden, die entsprechend kompatible Struktureinheiten aufweisen. Durch die Kenntnis der Struktur des Liganden lassen sich so unter anderem Rückschlüsse auf die mögliche Struktur bzw. auf spezielle Strukturelemente des Rezeptors ziehen.

In der Literatur wird der Begriff Ligand nicht einheitlich verwendet. Daher sollte betont werden, daß im Rahmen der vorliegenden Erfindung die Bezeichnung "Ligand" insbesondere für sogenannte "kleine Moleküle" verwendet wird, die bevorzugt kovalent an die Bindeoberfläche gebunden sind. Als Beispiele für solche Liganden können Oligomere oder auch kleine organische Moleküle angeführt werden, wie Peptide, Oligonucleotide, Kohlenhydrate (Glycoside), Isoprenoide, oder Lipidstrukturen. In der Literatur stellt zumeist das Molekulargewicht die Definitionsgrundlage für solche kleinen Moleküle dar.

Die mercaptophile Kopfgruppe M sollte bevorzugt zur Immobilisierung eines solchen Liganden, etwa eines sogenannten "Kleinen Moleküls (small molecule)" oder "niedermolekularem Molekül (low molecular weight molecule)" dienen.

In WO 89/03041 und WO 89/03042 werden Moleküle mit Molekülmassen von bis 7000 g/mol als kleine Moleküle beschrieben. Üblicherweise werden jedoch Molekülmassen zwischen 50 und 3000 g/mol, häufiger aber zwischen 75 und 2000 g/mol und meistens im Bereich zwischen 100-1000 g/mol angegeben. Als Beispiele sei auf die Schriften WO 86/02736, WO 97/31269, US-A-5928868, US-A-5242902, US-A-5468651, US-A-5547853, US-A-5616562, US-A-5641690, US-A-4956303 und US-A-5928643 verwiesen.

Im Rahmen der vorliegenden Erfindung soll das Molekulargewicht eines Liganden/kleines Molekül (ohne Anker und ohne Zusatzmolekül) zwischen 50 und 3000 g/mol, bevorzugt zwischen 75 und 1500 g/mol liegen.

Durch ihre charakteristische räumlichen bzw. elektronischen Strukturen, wie sie beispielsweise durch die Funktionalität einer Aminosäure, eines Nukleosides, einer heterocyclischen Verbindung, einer alicyclischen Verbindung, einer aromatischen Verbindung, eines Terpens, einer phosphororganischen Verbindung, eines Chelatkomplexes, eines Neurotransmitters, eines substituierten Amins, eines Alkohols, eines Esters, eines Ethers oder einer Carbonsäure sowie deren Derivate und Kombinationen daraus hervorgerufen werden, vermögen Liganden gegebenenfalls spezifisch an die Rezeptoren zu binden. Sie können unter Verwendung literaturbekannter Reaktionen (siehe z.B. J.S. Früchtel, G. Jung, Angew. Chem. Int Ed. 35, (1996) 17-42) synthetisiert werden. Diese Liganden müssen gegebenenfalls chemisch so modifiziert werden, dass sie mit der mercaptophilen Kopfgruppe der erfindungsgemäßen Anker reagieren können. Eine Auflistung beispielhafter Verbindungen die als Liganden, gegebenenfalls nach Modifikation einer funktionellen Gruppe oder Hinzufügen eines Zusatzmoleküls, mit den erfindungsgemäßen Bindeoberflächen verknüpft werden können findet sich in WO 00/73796 A2.

Unter dem Begriff "immobilisierter" oder "zu immobilisierender Interaktionspartner" werden neben Liganden jedoch auch Makromoleküle, vorzugsweise Biomoleküle oder Rezeptoren, wie Proteine, Peptide, wie Oligo- oder Polypeptide, DNA, RNA, Oligo- oder-Polynueleotide, prosthetische Gruppen, Vitamine, Lipide, Isoprenoide, Enzyme, Antikörper, Peptidhormone, Zytokine, Antibiotika, Kohlehydrate, wie z.B. Mono-, Oligo- und Polysaccharide, sowie deren Modifikationen, auch synthetische Moleküle wie z.B. Fusionsproteine und synthetisierte Primer verstanden. Auch diese Makromoleküle müssen zum Einsatz im Rahmen der vorliegenden Erfindung gegegebenenfalls chemisch modifiziert werden um ihre kovalente Anbindung an die mercaptophile Kopfgruppe der Ankermoleküle zu ermöglichen.

Als freie Interaktionspartner fungierende Moleküle sind bevorzugt in biologischen Systemen auftretende oder mit diesen interagierende Moleküle, insbesondere Rezeptoren, wie Proteine, DNA, RNA, Oligonucleotide, prosthetische Gruppen, Vitamine, Lipide, Mono-, Oligo- und Polysaccharide, aber auch synthetische Moleküle wie z.B. Fusionsproteine und synthetisierte Primer.

Zur Herstellung einer Meßoberfläche wird eine Lösung, die den zu immobilisierenden Interaktionspartner enthält, auf die Bindeoberfläche gebracht. Dabei können eine Vielzahl gleicher oder unterschiedlicher thioltragender oder mittels einer Thiolgruppe modifizierter biospezifischer Interaktionspartner kovalent an die Bindeoberfläche gebunden werden. Ein Vorteil der vorliegenden Erfindung liegt darin, dass die Konzentration des Liganden (bei Verwendung eines solchen) auf der Oberfläche ausschließlich von der Verdünnung der Bindeoberfläche bestimmt wird und nicht von der Konzentration des Liganden in der aufzubringenden Flüssigkeit. Das ist besonders dann ein Vorteil, wenn viele zu immobilisierende Interaktionspartner parallel verarbeitet werden, von denen nur eine ungefähre Konzentration bekannt ist, wie dies häufig bei Liganden der Fall ist, die aus der kombinatorischen Synthese erhalten werden. Damit erhöht sich die Reproduzierbarkeit und Vergleichbarkeit verschiedener Testmessungen.

Ausgehend von der erfindungsgemäßen Bindeoberfläche ist somit zur Herstellung der biospezifischen Meßoberfläche nur ein Schritt notwendig, nämlich die Kontaktierung mit dem thioltragenden Liganden. Dabei sollte der Ligand in einem geringen Überschuss vorliegen, um die quantitative Umsetzung zu gewährleisten. Ein weiterer Vorteil der Erfindung liegt in der somit geringen benötigten Menge an Ligand bei der Herstellung einer bioaktiven Oberfläche, insbesondere im Vergleich zu Einstufenveifahren.

Das Aufbringen des zu immobilisierenden Interaktionspartners beschränkt sich nicht auf spezielle Verfahren, jedoch können zur genaueren Lokalisierung der aktiven Stellen auf der Bindeoberfläche herkömmliche Pipettier- oder Tüpfelvorrichtungen, aber auch Stempel- oder Ink-Jet-Verfahren verwendet werden.

Ein weiterer Aspekt der Erfindung betrifft die Bereitstellung eines Arrays mit einer Vielzahl von Feldern auf einem planaren Festphasenträger. Jedes einzelne dieser Felder kann dabei als separate Meßoberfläche genutzt werden. Bevorzugt unterscheiden sich diese Meßoberflächen jeweils in der Art bzw. Struktur der darauf immobilisierten Interaktionspartner, wobei eine einzelne Meßoberfläche sowohl einen einzigen Typ eines immobilisierten Interaktionspartners als auch Mischungen unterschiedlicher Interaktionspartner präsentieren kann. Hierzu wird eine Vielzahl zu immobilisierender Interaktionspartner auf die Bindeoberfläche gebracht und einem Meßverfahren zugeführt. Bevorzugt geschieht dies, indem Lösungen der Interaktionspartner auf definierte, räumlich begrenzte Bereiche der Bindeoberfläche aufgebracht werden, beispielsweise mittels einer Pipettiervorrichtung, einer Tüpfelvorrichtung, einer Mikropipettiervorrichtung oder eines Ink-Jet-Verfahrens.

Die räumliche Struktur des entstehenden Arrays kann durch eine mechanische Strukturierung des Trägers vorgegeben werden. Werden in der vorliegenden Erfindung strukturierte Trägerplatten als Sensoroberflächen eingesetzt, so weisen diese daher bevorzugt eine Vielzahl regelmäßig angeordneter, positionsadressierbarer Felder zur Bereitstellung von Bindeoberflächen auf, wobei diese Felder in Kavitäten mit nur geringer Tiefe lokalisiert sind. Damit wird eine Flüssigkeitsbarriere bereitgestellt, wobei gleichzeitig die Oberfläche so gering wie möglich gehalten wird, um eventuelle unspezifische Adsorptionsphänomene zu minimieren. Weiterhin beinhalten diese Felder eine Schicht des Materials, das die Anbindung der thiolfunktionalisierten Anker ermöglicht. Vorzugsweise besitzen die Kavitäten eine Tiefe von 20-100 µm, und die Anker werden auf ihrem Boden immobilisiert, der dann z.B. von einem Metall oder Metalloxid, bevorzugt von einem Edelmetall wie Gold gebildet wird.

Mit Hilfe so ausgestalteter Felder gelingt es, Nachteile bezüglich der unspezifischen Bindung sowie des Übersprechens zu vermeiden oder zu minimieren. Zudem kann die Herstellung einer solchen Trägerplatte dadurch kostengünstig gestaltet werden, daß Verfahren und Materialien der Photolithographie und Ätztechniken, wie sie in der Halbleitertechnik angewendet werden, zum Einsatz kommen.

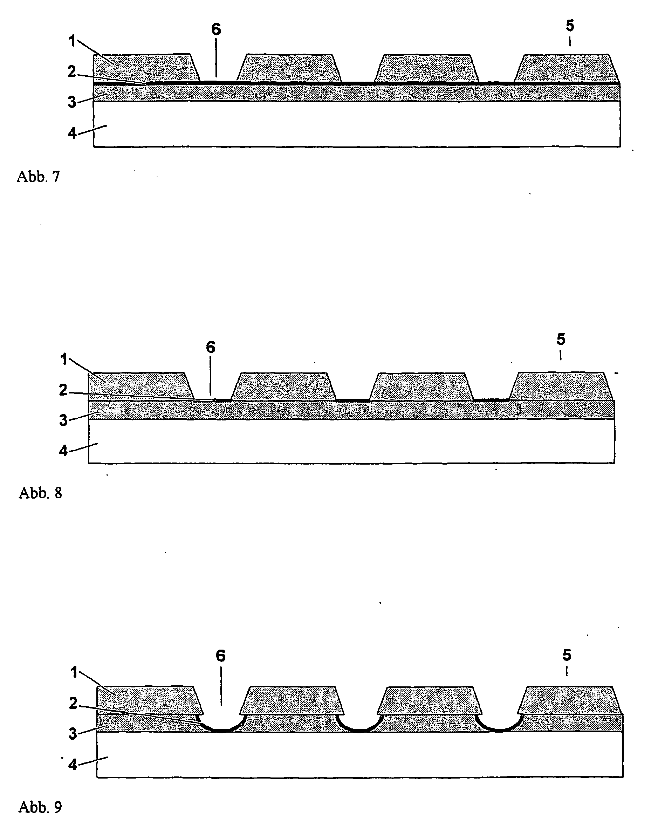

Im folgenden werden bevorzugte Ausführungsformen einer solchen Trägerplatte unter Bezugnahme auf die Abbildungen näher erläutert. Dabei zeigen Abb. 7 bis 9 jeweils einen schematischen Ausschnitt bevorzugter Trägerplatten im Querschnitt.

Zur Herstellung einer Trägerplatte (5) nach Abb. 7 kann von einem kupferkaschierten Basismaterial (4) ausgegangen werden, das bevorzugt bereits eine Metallschicht (3), wie Kupfer, trägt und welches in einem galvanischen Abscheideprozeß mit besagter Trägerschicht (2) versehen wird. Die Dicke der Trägerschicht beträgt nur wenige Mikrometer, genau die notwendige Stärke, um eine geschlossene Schicht herzustellen. Nach der Galvanik wird die Platte mit einer UV-belichtbaren Schutzschicht (1) versehen. Dafür können entweder in der Halbleiterfertigung übliche Photoresists oder andere UVbelichtbare und damit strukturierbare Schutzlacke verwendet werden. Die verwendeten Lackschichten weisen vorzugsweise Dicken von 20 µm - 100 µm auf. In einem Belichtungsschritt wird ein Bild einer Maske in die Schutzschicht projiziert. Die Maske weist vorzugsweise runde oder rechteckige/quadratische Muster auf. Nach einem Entwicklungsschritt entstehen in der Schutzschicht definierte Öffnungen, die die darunterliegende Trägerschicht freilegen. Die photostrukturierbare Schutzschicht bildet somit nach der Strukturierung gleichzeitig die Wandung der Kavitäten (6) und somit die Form der Kavität (6) und deren Öffnung.

Wird das Aufbringen und Strukturieren der Schutzschicht (1) dem Aufbringen der Trägerschicht (2) vorangestellt, so erhält man einen Träger (5) gemäß Abb. 8.

Abb. 8 zeigt eine Trägerplatte (5) mit tieferen Kavitäten (6), wobei jedoch der Anteil an ungeschützter Wandfläche nicht erhöht wird. Diese Trägerplatte weist bevorzugt ein Basismaterial (4) auf, auf dessen Oberfläche eine metallische Beschichtung (3) vorgesehen ist, die wiederum mit einer Schutzschicht (1) versehen ist, wobei in der Schutzschicht (1) und der Metallschicht (3) mindestens eine Kavität (6) ausgebildet ist, die im Bereich der Metallschicht (3) muldenförmig ist und mit einer Trägerschicht (2) versehen ist und die im Bereich der Schutzschicht (1) in Richtung zur Mulde konisch verjüngend ausgebildet ist, wobei der untere Rand des in der Schutzschicht (1) vorgesehenen Kavitätenabschnitts einen kleineren Durchmesser aufweist als der obere Rand des in der Metallschicht (3) ausgebildeten Kavitätenabschnitts.

Die Herstellung einer Trägerplatte wie sie in Abb. 9 gezeigt ist geht ebenfalls von einer beschichteten Platte aus. In diesem Fall ist jedoch die Dicke der schon vorhandenen Schicht (3) vorzugsweise 100 µm - 150 µm. Mittels eines Photoresists (in der Abbildung nicht dargestellt) wird die Schicht (3) so strukturiert, daß diese bereits Vertiefungen aufweist. Diese Platte wird im Anschluß galvanisch mit der Trägerschicht (2) beschichtet. In einem zweiten Photolithographieschritt wird dann ebenfalls eine Schutzschicht (1) so strukturiert, daß sich über den in Schicht (3) geätzten Kavitäten (6) eine Struktur in der Schutzschicht (1) ausbildet. Die Tiefe der gebildeten Kavität (6) setzt sich hier aus der Tiefe der geätzten Struktur und der Dicke der Schutzschicht (1) zusammen.

Bevorzugt sind die Kavitäten derart angeordnet, daß auf der Trägerplatte ein regelmäßiges, vorzugsweise kartesisches Raster von Spalten und Streifen entsteht. Die Größe und Form der Trägerplatte kann beliebig gewählt und leicht an das verwendete Detektionssystem angepaßt werden. Bei Verwendung von Tüpfelrobotern für die Immobilisierung der Anker oder der Interaktionspartner, oder bei Vorliegen der Bindeoberfläche auf Mikrotiterplatten, ist der Abstand der Felder zueinander bevorzugt dem verwendeten Mikrotiterformat bzw. der verwendeten Tüpfeleinrichtung anzupassen. Die Anzahl der Felder kann die Anzahl der Untereinheiten der Mikrotiterplatte auch überschreiten, d.h. es kann ein Vielfaches an Feldern pro Fläche verwendet werden. So kann beispielsweise eine quadratische Trägerplatte mit einer Seitenlänge von ca. 12 cm insgesamt 9216 Felder besitzen, die mittels eines Pipettierroboters aus sechs konventionellen 1536er Mikrotiterplatten belegt werden können.

Eine räumlich strukturierte Präsentation gleicher oder unterschiedlicher immobilisierter Interaktionspartner kann jedoch auch durch kovalente Bindung des Interaktionspartners auf einem vorgegebenen Abschnitt der Bindeoberfläche nach gezieltem Aufbringen eines Flüssigkeitsvolumens erreicht werden, ohne dass dazu eine physikalische Unterteilung in einzelne Kompartimente nötig ist. Dazu lassen sich beispielsweise Techniken, wie sie in der EP-A-0 872 735 zum Aufbringen von Reagenzspots auf Metall- oder Metalloxidoberflächen beschrieben sind in analoger Weise auf die Bindeoberflächen der vorliegenden Erfindung anwenden. Werden Lösungen der Interaktionspartner/Liganden auf eine homogene Bindeoberfläche aufgebracht, so wird die Konzentration des Liganden/Interaktionspartners auf der fertien Meßoberfläche wird durch die Verdünnung der Bindeoberfläche bestimmt. Damit ergibt sich gegenüber EP 872 735 A1 der zusätzliche Vorteil, daß zur Erzeugung eines Meßarrays nur einmal genau definierte Flüssigkeitsmengen auf die Festphasenträgeroberfläche aufgebracht werden müssen (d.h. bei der Generierung der Bindeschicht).

Die vorliegende Erfindung kann im HTS, bei der Wirkstoffsuche oder im medizinisch diagnostischen Bereich eingesetzt werden. Geeignete Meßmethoden zur Detektion von Interaktionen zwischen immobilisiertem (oberflächengebundenem) Interaktionspartner/Ligand und freiem Interaktionspartner/Rezeptor, bei denen der Festphasenträger ausschließlich der Immobilisierung eines Interaktionspartners dient, beruhen auf dem Nachweis der spezifischen Bindungsreaktion mittels elektrochemischer (Elektro-Immunoassays), radiochemischer (z. B. Radioimmunoassays), massensensitiver oder optischer Verfahren wie Fluoreszenz- oder Lumineszenzmessungen, insbesondere Enzymassays. Für letztere ist die so genannte ELISA-Technik (enzyme-linked immunosorbent assay, Immunoassays unter Verwendung der Festphasentechnik) bevorzugt.

Zur markierungsfreien Detektion der Interaktionen sind reflexionsoptische Verfahren, wie die Oberflächenplasmonenresonanz, geeignet. Hierbei ist der Festphasenträger Bestandteil des Sensorsystems.

Aufgrund ihrer vorstehend beschriebenen Vorteile können die erfindungsgemäßen Oberflächen jedoch auch in klassischen Verfahren, wie der Affnitätschromatographie, Anwendung finden.

### BEISPIELE

### Beispiel 1 Synthese einer Verdünnerkomponente

a) Immobilisierung von N-(N⁵-Fmoc-5-aminopentyl)-11-mercaptoundecanamid an Chlortrityl-Harz 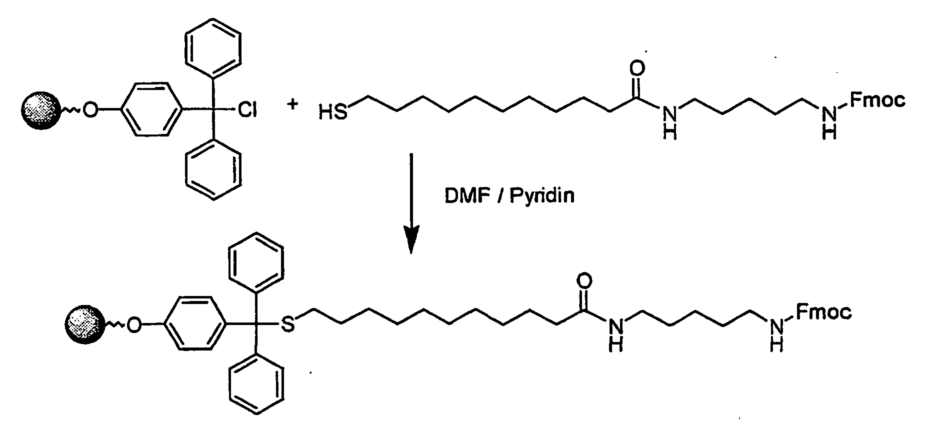 600 mg (1,15 mmol) N-(N⁵-Fmoc-aminopentyl)-11-mercaptoundecanamid, erhältlich aus S-geschütztem 11-Mercaptoundecanamid und Fmoc-1,5-diaminopentanhydrochlorid, wurden in 15 ml DMF gelöst und mit 2 g Methoxylrityl-chlorid-Harz (1,6 mmol) (Novabiochem) versetzt. Die Suspension wurde 1 h vorsichtig geschüttelt. Anschliessend wurden 500 µl Pyridin zugegeben und die Suspension weitere 3 h geschüttelt. Das Harz wurde anschliessend 1 x mal mit N,N-Dimethylformamid (DMF), 1 x mit 5% Wasser in DMF, 4 x mit DMF, 3 x mit Dichlormethan und 2 x mit Hexan gewaschen und im Vakuum getrocknet. Die Beladung des Harzes mit N-(N⁵-Fmoc-5-arninopentyl)-11-mercaptoundecanamid wurde über Fmoc-Analytik (G.B. Fields, R.L. Noble, Int. J. Peptide Protein Res. 1990, 35, 161-214) zu 0,35 mmol/g bestimmt (Ausbeute 60 % d.Th.).
b) Allgemeine Vorschrift für die Kupplung von Fmoc-8-Amino-3,6-dioxa-octansäure (Fmoc-Ado) 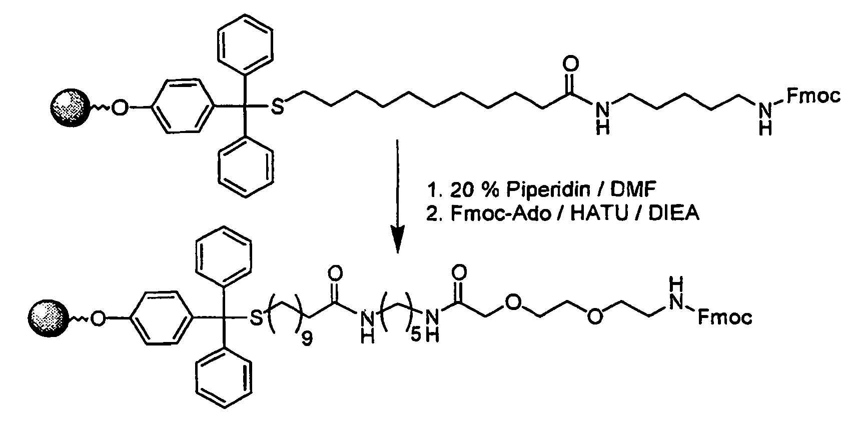 Für die Abspaltung der Fmoc-Schutzgruppe wurde 1 g des beladenen Harzes (0,35 mmol) 20 min in 15 ml 1/3 (v/v) Piperidin/DMF vorsichtig gerührt und anschliessend 6 mal mit DMF gewaschen. Die Kupplung von Fmoc-8-amino-3,6-dioxa-octansäure erfolgte durch 4 h Inkubation des Harzes mit einer Lösung von 270 mg (0,70 mmol) Fmoc-8-amino-3,6-dioxa-octansäure, 270 mg (0,71 mmol) O-(7-Azabenzotriazol-1-yl)-N,N,N,N'-tetramethyluroniumhexafluorophosphat (HATU) und 250 µl (1,44 mmol) Ethyldiisopropylamin (DIEA) in 7 ml DMF. Anschliessend wurde das Harz 5 mal mit DMF, 3 mal mit Dichlormethan und 2 mal mit Hexan gewaschen und getrocknet.
c) Synthese einer Verdünnerkomponente 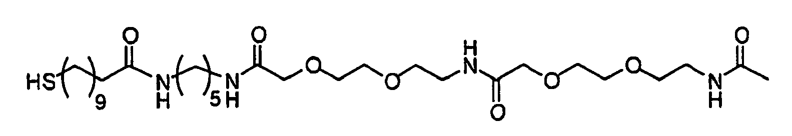 An 500 mg Harz aus b) (0,175 mmol) wurde wie unter b) beschrieben Fmoc-8-amino-3,6-dioxa-octansäure gekuppelt und anschliessend die Fmoc-Schutzgruppe wie unter b) beschrieben abgespalten. Anschliessend wurden die freien Aminogruppen durch 30 min Inkubation des Harzes mit 10 ml 1/1/2 (v/v/v) Essigsäureanhydrid/Pyridin/DMF acetyliert. Dann wurde das Harz 5 mal mit DMF und 3 mal mit Dichlormethan gewaschen. Die Abspaltung des Produktes vom Harz erfolgte mit 2/18/1 (v/v/v) Trifluoressigsäure /Dichlormethan / Triethylsilan. Das Produkt wurde durch präparative RP-HPLC gereinigt und mittels LC/MS analysiert.
   LC-MS (ber.): [M+H]⁺ 635,5 (635,4), [M+Na]⁺ 657,5 (657,4)

### Beispiel 2 Synthese einer Ankerkomponente

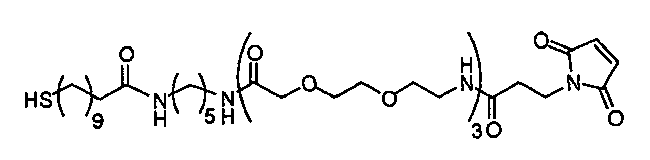

An 100 mg Harz des in Beispiel 1b) dargestellten Harzes wurde wie unter 1b) beschrieben zweimal Fmoc-8-amino-3,6-dioxa-octansäure gekuppelt und anschliessend die Fmoc-Schutzgruppe abgespalten. Anschliessend wurde 3-Maleinimidopropansäure durch 1 h Inkubation des Harzes mit 4 eq. 3-Maleinimidopropansäure und 4 eq. Diisopropylcarbodiimid in DMF (c = 0,15 M) gekuppelt.

Dann wurde das Harz 5 mal mit DMF und 3 mal mit Dichlormethan gewaschen. Die Abspaltung des Produktes vom Harz erfolgte mit 2/18/1 (v/v/v) Trifluoressigsäure /Dichlormethan / Triethylsilan. Das Produkt wurde durch präparative RP-HPLC gereinigt und mittels LC/MS analysiert.
LC-MS (ber.): [M+H]⁺ 889,2 (889,5) [M+Na]⁺ 911,1 (911,5)

### Beispiel 3 Herstellung einer Bindeoberfläche

Die Bindeoberfläche setzt sich aus der Ankerkomponenten aus Beispiel 2 und der Verdünnerkomponenten aus Beispiel 1 zusammen.
Zunächst werden Stammlösungen der Einzelkomponenten durch Auflösen der Feststoffe in Ethylenglycol / 0,1 % TFA mit der gewünschten Molarität hergestellt, die im Ellman-Test (G.L. Ellman, Arch. Biochem. Biophys. 82 (1959), 70-77) oder über den Extinktionskoeffizienten bei 295 nm überprüft wird, hergestellt. Anschließend werden die Stammlösungen von Anker- und Verdünnerkomponente im gewünschten Verhältnis (1:10, v/v) gemischt und die Goldoberfläche mit dieser 100µM bis 1 mM Lösung 1 h bei Raumtemperatur inkubiert, anschliessend mit Methanol / 0,1 % TFA sowie mehrfach mit Wasser / Essigsäure (2ppm) gewaschen und im Stickstoffstrom getrocknet.

### Beispiel 4 Immobilisierung eines Liganden ("kleinen Moleküls")

Die Ankopplung von Ac-p-Tyr-Cys-NH₂ an die Bindeoberfläche aus Beispiel 3 erfolgt durch 15-30 minütige Inkubation einer 10-100 µM Lösung der Verbindung. Danach wird die Oberfläche mit Essigsäure (2 ppm) in Wasser gespült und getrocknet. Anschließend wird mit Essigsäure (2 ppm) in Wasser sowie mit Isopropanol gewaschen und die Oberfläche im Stickstoffstrom getrocknet.

### Beispiel 5 Immobilisierung von Proteinen auf Biacore® J1 Chip

Die Präparation der Proteinoberfläche erfolgt durch Inkubation der Bindeoberfläche aus Beispiel 3 (1:10 Verdünnung) mit einer 17 µM Lösung von Caspase7T7 (durch ein T7 und ein oligo-His-tag modifiziert) in 0.2 M NaH₂PO₄/Na₂HPO₄, pH 7, (20 Min., Raumtemperatur). Anschliessend wird der Chip mit Essigsäure/Wasser (1 µl/500 ml) gewaschen, 5 Min. mit 10 mM 2-Mercaptoethanol in 0.2 M NaH₂PO₄/Na₂HPO₄, pH 7, behandelt, mit Essigsäure/Wasser (s.o.) gewaschen und im Stickstoffstrom getrocknet.
Der Erfolg der Proteinimmobilisierung wird mittels SPR am Biacore® 3000 durch Aufspritzen einer 6.7 nM Lösung (HBS, pH 7.4) eines anti-T7-Antikörpers (Novagen) bzw. einer 11 nM Lösung (HBS, pH 7.4) eines anti-oligo-Histidin-Antikörpers (Sigma) überprüft.
Die Änderung des SPR-Signals (in RUs) nach Aufspritzen von 100 µl Antikörperlösung (Flussrate: 10 µl/min, Laufpuffer: HBS, pH 7.4) beträgt:

| Antikörper | RU |
|---|---|
| anti-T7-Antikörper, 6.7 nM | 206 |
| anti-oligo-His-Antikörper, 11 nM | 1067 |

### Beispiel 6 Grazing-angle FTIR

Ziel dieser Meßmethode ist die Schwingungs-Spektroskopie einer dünnen Schicht, die sich auf einer Metall-Oberfläche befindet. Dabei wird das IR-Licht unter streifendem Einfall an der Metall-Oberfläche reflektiert. Die Messungen erfolgen ausschließlich mit in Einfallsebene polarisiertem IR-Licht, da Licht mit der Polarisationsrichtung senkrecht dazu nicht zur Messung beitragen kann. Außerdem tragen nur die Dipolübergangsmomente zur Absorption bei, die zumindest parallele Anteile zum E-Feld der Strahlung besitzen.
Wenn die auf der Oberfläche befindlichen Moleküle nun orientiert sind bedeutet dies zusätzliche Auswahlregeln für das Auftauchen der Bande im Spektrum. Die Empfindlichkeit dieser Meßmethode reicht nun aus, um IR-Spektren selbst monomolek-ular dünner Filme messen zu können, wobei typischerweise Absorptionen von einigen mOD erhalten werden. Für die folgenden Messungen wurde ein kommerzielles FTIR Gerät (Bio-Rad FTIR Spektrometer Model FTS 175C) benutzt.

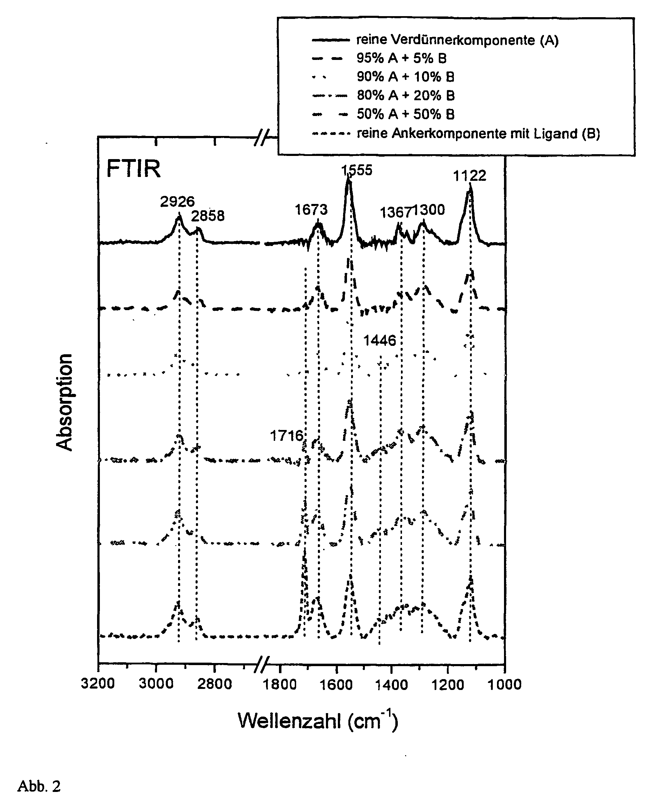

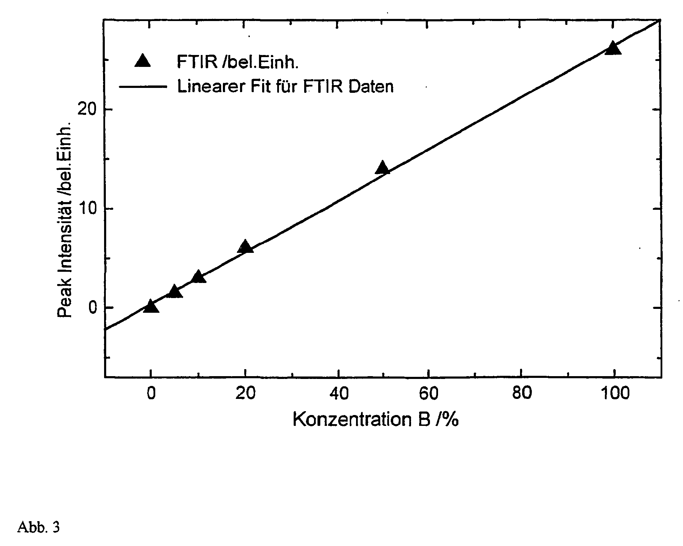

Die obere Meßkurve in Abb. 2 zeigt das FTIR Spektrum der reinen Verdünnerkomponente (A). Man erkennt die CH₂ Banden im Bereich von 2900cm⁻¹ CONH Banden im Bereich 1500-1700 cm⁻¹ und die C-O-C Bande bei 1122cm⁻¹. Die unterste Meßkurve zeigt das Spektrum der reinen Ankerkomponente mit Ligand (B). Verglichen mit dem Spektrum des Verdünners fällt die Bande bei 1716cm⁻¹ auf. Diese kann der Imidgruppe der Ankerkomponente zugeordnet werden. Bei den Oberflächen, die mit wachsender Konzentration der Ankerkomponente hergestellt wurden (Spektren von oben nach unten) kann das systematische Anwachsen der Imidbande beobachtet werden. Zur quantitativen Erfassung wurde die Fläche des Peaks bei 1716cm⁻¹ ermittelt und gegen die Konzentration der Ankerkomponente aufgetragen (siehe Abb. 3-). Eine lineare Anpassung an die Meßdaten verdeutlicht den Zusammenhang und damit die Übereinstimmung der Konzentration der Ankermoleküle auf der Goldoberfläche mit der Konzentration in der Lösung während der Goldbeschichtung.

### Beispiel 7 XPS und Ellipsometrie Messungen

Bei der Röntgen- Photoelektronen Spektroskopie (XPS) wird die kinetische Energie der Elektronen nachgewiesen, die nach der Interaktion von Atomen mit monoenergetischen Röntgenstrahlen nach dem Photoelektrischen Effekt entstehen. Als Röntgenquelle wurde hier die MgK_{α1,1} Linie (h=1253.6eV) benutzt, die unter einem Winkel von 45° auf die zu untersuchende Oberfläche eingestrahlt wurde. Der Detektor mißt die Anzahl der ebenfalls unter 45° austretenden Elektronen, klassifiziert nach seiner Energie. In dem erhaltenen Energiespektrum gibt es Peaks bei Energien, die gerade der Differenz aus der Energie der eingestrahlten Röntgenstrahlung und der Bindungsenergie des Elektrons im Atom entspricht. Diese Bindungsenergien sind spezifisch für jedes Atom und man kann daher die Zusammensetzung der Moleküle auf der Oberfläche bestimmen. Besonders groß ist der Peak der von den Au4f Elektronen herrührt. Da das Gold aus der Sicht des Detektors sich hinter der Moleküllage befindet werden die von dem Gold abgelösten Elektronen wieder von der Molekülschicht absorbiert. Daher kann man aus dem Au4f-Peak der Probe (Iₛₐₘₚₗₑ) und dem entsprechenden Peak einer reinen Goldoberfläche (I₀) die Schichtdicke d der Probe bestimmt werden, nach dem Zusammenhang *d =* λ(ln *I*₀)/ln(*Iₛₐₘₚₗₑ*). Dabei ist λ=37Å die mittlere freie Weglänge der 1169.6 eV Elektronen in der Schicht.

In der Ellipsometrie wird die Änderung des Polarisationszustandes des Lichtes gemessen, wenn es an dem System aus Molekülschicht, Goldschicht und Substrat reflektiert wird. An jeder Grenzfläche dieses Systems gibt es entsprechend den Fresnelschen Formeln einen reflektierten und einen transmittierten Lichtstrahl. Da die Schichten sehr dünn sind, tritt innerhalb der Molekül- und der Goldschicht Vielfachreflexion ein, d.h. die Amplituden der Lichtstrahlen müssen phasenrichtig aufsummiert werden. Als Ergebnis dieser Summation erhält man einen Reflexionskoeffizienten für die Lichtkomponente, die in der Einfallsebene polarisiert ist und einen zweiten für die Komponente die senkrecht dazu polarisiert ist. In diesen beiden mathematischen Ausdrücken sind als Parameter die Schichtdicken und die (komplexen) Brechungsindices der Materialien enthalten. Diese beiden Reflexionskoeffizienten werden von einem spektroskopischen Ellipsometer (hier J.A. Woollam Co., Inc. M-44^{®}) bestimmt, und dies zusätzlich wellenlängenabhängig. In einem Fit der gemessenen wellenlängen-abhängigen Reflexionskoeffizienten kann dann, unter Vorgabe des Brechungsindex der Moleküllage (hier 1,45), die Dicke derselbigen erhalten werden.

Die aus XPS-Messungen und Ellipsometrie Messungen erhaltenen Schichtdicken stimmen mit der Länge des Verdünnermoleküls aus Beispiel 1 überein. In gestreckter Konfiguration beträgt die Moleküllänge der Verdünnerkomponente 44 Å. Da die Moleküle nicht senkrecht von der Oberfläche abstehen, sondern i.d.R. unter einem spezifischen Winkel gebunden sind, paßt die etwas kürzere gemessene Schichtdiche (34Å) gut mit der Vorstellung zusammen, daß es sich bei diesen Oberflächen um einen SAM handelt. Dann wäre ein Bindungswinkel der Moleküle von ca. 50° zur Oberfläche vorstellbar.

### Beispiel 8 Unspezifische Proteinadsorptionsresistenz verschiedener Verdünner

Die Proteinadsorptionsresistenz wurde mittels Oberflächenplasmonresonanz am Biacore® 3000 mit folgenden Proteinen bestimmt:

| | |
|---|---|
| Ribonuclease A (Rind) : | 0.1 mg/ml |
| Lysozym (Huhn) : | 0.1 mg/ml |
| Ovalbumin (Huhn) : | 0.1 mg/ml |
| D-Aminosäure-Oxidase (Schwein) : | 0.1 mg/ml |
| Pyruvat-Kinase (Kaninchen) : | 0.1 mg/ml |
| Fibrinogen (human) : | 1 mg/ml |
| Glutathion-Reduktase (Weizenkeim) : | 1 mg/ml |

Zur Ermittlung der Proteinadsorptionsresistenz wurden alle Proteine in HBS pH 7.4 in den oben angegebenen Konzentrationen gelöst, jeweils 100 µl wurden am Biacore® 3000 (Flussrate: 20 ul/min, Laufpuffer: HBS pH 7.4) auf die entsprechenden Oberflächen aufgespritzt und die Änderung des SPR-Signals (Response Units = RU) gemessen. Ein Zuwachs von 1000 RU entspricht einer Proteinmenge von 1 ng/mm².

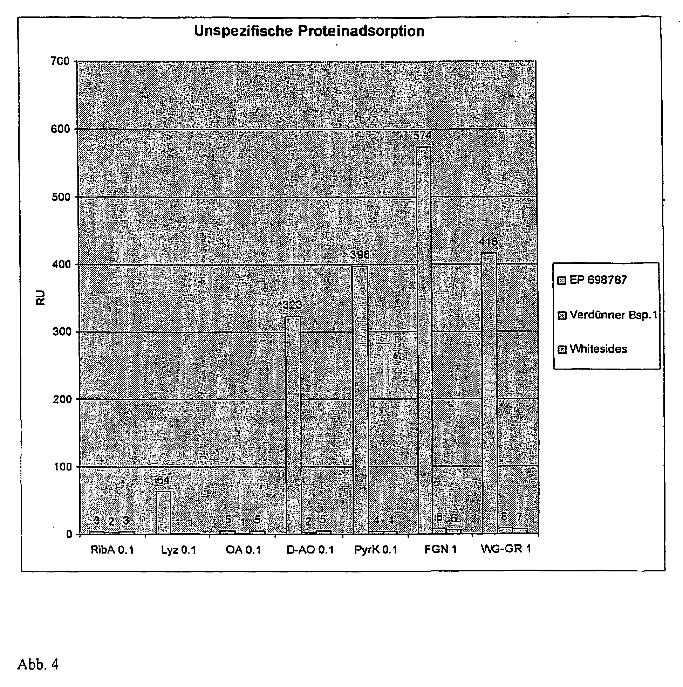

Die gezeigten Werte in Abbildung 4 sind Mittelwerte aus 4 Messungen.

Als Verdünner wurden ein kurzkettiges Disulfid (Verbindung A aus EP 698 787), das Verdünnermolekül aus Beispiel 1 und 12,15,18-Trioxa-20-hydroxyicosan-1-thiol (Whitesides et al. J.Am.Chem.Soc. 1995, 117, 12009-10) verwendet. Wie die Ergebnisse zeigen, hat die Kettenlänge des Moleküls einen Einfluß auf die unspezifische Adsorptionsresistenz, die demnach bei kurzkettigen Verdünnermolekülen verstärkt auftritt.

### Beispiel 9 Quantitative Umsetzung von Phosphotyrosin (pY) an der Bindeoberfläche

Zur Anbindung von pY an die Bindeoberfläche aus Beispiel 3 wird analog zu Beispiel 4 pY mit Cystein als Zusatzmolekül verwendet ("pY-Tag").

Ein Goldchip wurde mit einer 1:10 Mischung der Ankerkomponente aus Beispiel 2 und der Verdünnerkomponente aus Beispiel 1 in Ethylenglycol und 1% TFA in einer Gesamtkonzentration von 0,5 mM inkubiert. Anschließend wurde der Chip mehrfach in Methanol / 0,1%TFA und daraufhin in Wasser pH 7,0 gewaschen. So vorbehandelte Chips wurden im Stickstoffstrom getrocknet.

Um zu testen, in welchem Konzentrationsbereich eine komplette Abreaktion der präsentierten Maleimid-Funktionalitäten erfolgt und somit konstante Signalintensität der Lumineszenz zu beobachten ist, wurden 0,15 µl Volumen von pY-Tag in Verdünnungen von 10 µM, 1 µM, 100 nM und 10 nM aufgbracht. Für jede Konzentration wurden vierfache Bestimmungen durchgeführt. In den freien Bereichen wurden die Maleimid-Gruppen anschließend durch Inkubation des Chips in 0,2 M Phosphatpuffer pH 7.0, 10mM Mercaptoethanol für 30 min abgesättigt.

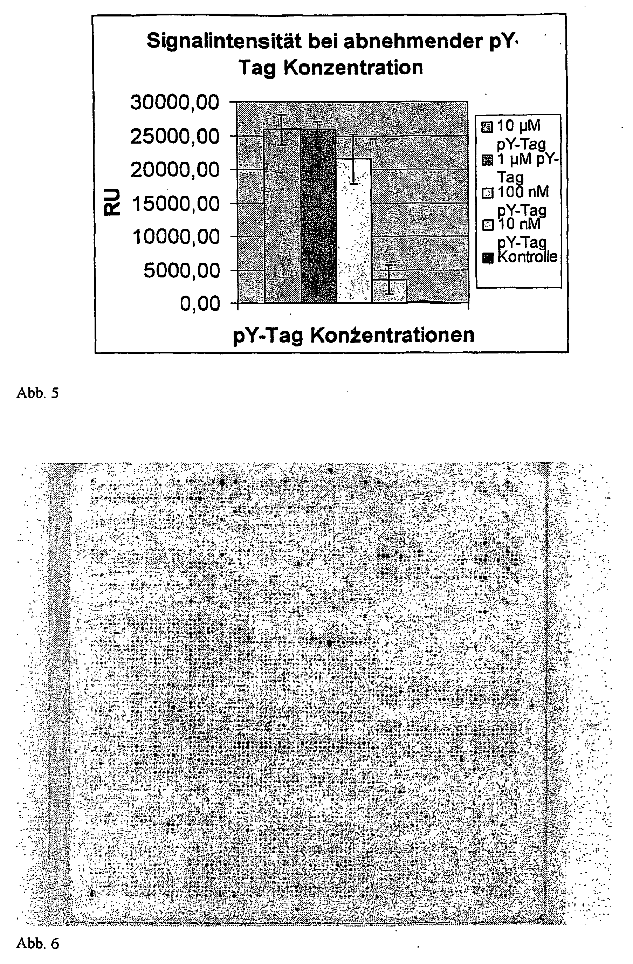

Solche Chips wurden anschließend über Nacht in BSA-Blocking (50 mM Tris/HCl, 150 mM NaCl, 5 g/l BSA, 0,05 % (v/v) Tween-20^{®}, pH 7,3) Lösung inkubiert. Der Nachweis der pY Gruppen auf der Oberfläche des Chips erfolgte durch einen Immunoassay bei dem der Chip zunächst mit einer 1:5000 a-pY-Antikörper in Blocking-Lösung und danach mit 1:5000 anti-mouse-Fab-POD inkubiert wurde. Zwischen den Inkubationsschritten wurde mit Blocking-lösung gewaschen (2 x 1 min). Vor der Detektion des Luminescence Signals mit dem Super-Signal- Plus Substrate von Roche Diagnostics, wurde noch einmal in TBS-Puffer gewachen. Die Luminescence Reaktion wurde in eine Lumi-Imager (Roche Diagnostics) verfolgt. Die Signalintensitäten wurden anschließend integriert und sind in der Abbildung aufgetragen. Die Standardabweichungen der vierfach Bestimmungen wurden berechnet und sind als Fehlerbalken Abbildung 5 zu entnehmen.

Als Ergebnis zeigt sich, daß die Siganlintensitäten innerhalb der Fehlergrenzen über einen Konzentrationsberreich von 3 Zehnerpotenzen konstant sind. Erst bei einer sehr starken Verdünnung des pY-Tags ist eine Signalabnahme zu erkennen. Die Konstanz des Signals ergibt sich aus der Tatsache, daß die Reaktion des Maleimides mit dem Thiol näherungsweise quanitativ erfolgt, solange das Thiol in einem bestimmten Überschuss vorhanden ist.

### Beispiel 10 Herstellung eines Arrays mit einer Vielzahl von Meßoberflächen

Ein Goldchip (40 nm Gold/l nm Chrom auf Glas) in den Abmaßen 12 x 12 cm wurde mit einer 1:10 Mischung (Verdünnung) von Ankerkomponente aus Beispiel 2 und der Verdünnerkomponente aus Beispiel 1, in Ethylenglycol und 1% Trifluoressigsäure (TFA) in einer Gesamtkonzentration von 1,0 mM inkubiert. Anschließend wurde der Chip mehrfach in Methanol/1%TFA und daraufhin in Wasser pH 7,0 gewaschen. So vorbehandelte Chips wurden im Stickstoffstrom getrocknet.

Auf diese Chips wurde dann eine Bibliothek von 9216 thiolhaltigen Liganden in einer Anordnung von 96 x 96 spots mit einem Spot/Spot -Abstand von 1,125 mm durch eine Tüpfelvorrichtung abgesetzt. Die thiolhaltigen Liganden, die auf die Oberfläche gebracht werden sind in einer 40 µM Lösung aus 0,2 M Pi, 5 mM EDTA und 10% (v/v) Ethylenglycol pH 7,0 gelöst. Die Tüpfelvorrichtung setzt ca. 10 nl pro spot ab, so daß in jedem Spot ein hoher Überschuß des thiolhaltigen Liganden gegenüber der Oberflächengebundenen Maleimid Gruppe gewährleistet wird und vollständiges Abreagieren der Maleimid Gruppen erreicht werden kann. In den freien Bereichen wurden die Maleimid Gruppen anschließend durch Inkubation des Chips in 0,2 M Phosphatpuffer pH 7.0,10mM 2-Mercaptoethanol für 30 min abgesättigt.

Solche Chips wurden anschließend über Nacht in BSA-Blocking (50 mM Tris/HCl, 150 mM NaCl, 5 g/l BSA, 0,05 % (v/v) Tween-20®, pH 7,3) Lösung inkubiert. Die Analyse potentieller Bindungspartner des Target Proteins Thrombin erfolgte im anschließenden Immunoassay.

Zunächst wurden hierzu der Chip 4 h in 10 nM Thrombin in Blocking Lösung inkubiert. Nach zweimaligem 2 minütigen Waschen in Blocking Lösung wurde eine 1:1000 Verdünnung eines polyklonalem anti Thrombin Antikörper für 2 h mit dem Chip inkubiert, nach erneutem zweimaligen Waschen in Blocking Lösung wurde ein anti-rabbit-Antikörper-POD Konjugat zum Nachweis der Bindungsereignisse für 2 h mit dem Chip inkubiert. Abschließend wurde der Chip zweimal für 2 min in TBST gewaschen. Die Chemilumienszenz Reaktion wurde durch Umsatz des Lumi-Light Plus Substrates im Lumi Imager (Roche Diagnostics, Deutschland) nachgewiesen. Abbildung 6 zeigt eine Aufnahme der Chemilumineszenzreaktion (10 nM Thrombin). Schwarze Punkte (im Imager als helle spots) weisen auf Bindung des Thrombins hin. Diskrete Intensitäten sind als Spots in bestimmten Positionen zu erkennen. Da jede Verbindung auf dem Array eine bestimmte räumliche Koordinate aufweist, lassen sich den Spots bestimmte chemischen Strukturen zuordnen.

Beispiel 11 Synthese weiterer Anker- und Verdünnerkomponenten

### 1) Aufbau weiterer Anker- und Verdünnerverbindungen (-komponenten) ausgehend von der Umsetzung 4-Methoxytrityl-geschützter Mercaptoundecansäure mit verschiedenen Diaminen.

Ansatz:
Jeweils:
1 g (2,04 mmol) S-(4-Methoxytrityl)-mercaptoundecansäure
462 mg (2,24 mmol) Dicyclohexylcarbodiimid (DCC)
236 mg (2,04 mmol) n-Hydroxysuccinimid (NHS)
   a) 755,3mg (850,6µl; 10,2 mmol) 1,3-Diaminopropan
   b) 1,51g (1,49ml; 10,2mmol) 1,8-Diamino-3,6-dioxaoctan
   c) 1,04g (1,19ml; 10,2mmol) 1,5-Diaminopentan

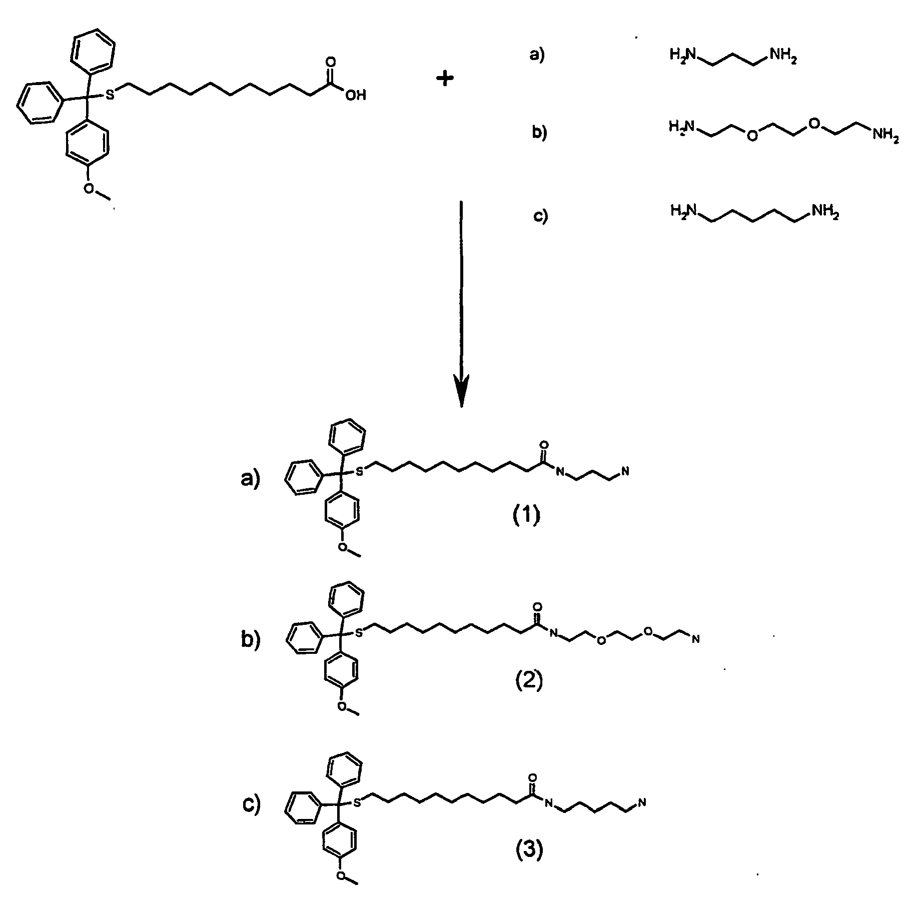

### Durchführung:

Jeweils 1g S-(4-Methoxytrityl)-mercaptoundecansäure, 462 mg (2,24 mmol) DCC und 236 mg (2,04 mmol) NHS wurden in jeweils 20ml Dichlormethan (DCM) gelöst und 30 min bei Raumtemperatur gerührt. Die drei Diamine a) bis c) wurden jeweils in 20ml DCM gelöst und unter Rühren der S-(4-Methoxytrityl)-mercaptoundecansäure-NHS-Ester in 10 Portionen über 30min zu den Diamin-Lösungen gegeben. Die Reaktionsgemische wurden 3h gerührt, der Harnstoff abfiltriert und das Lösungsmittel im Vakuum eingeengt. Die Produkte wurden in Ethylacetat gelöst und jeweils 3 mal mit 10%iger Natriumcarbonatlösung, 2 mal mit gesättigter Natriumchloridlösung und 2 mal mit destilliertem Wasser gewaschen. Die Lösungen wurde über Natriumsulfat getrocknet und am Rotationsverdampfer zur Trockene eingeengt.

### 2) Verlängerung der Amine (1) - (3) mit Trioxaundecansäure.

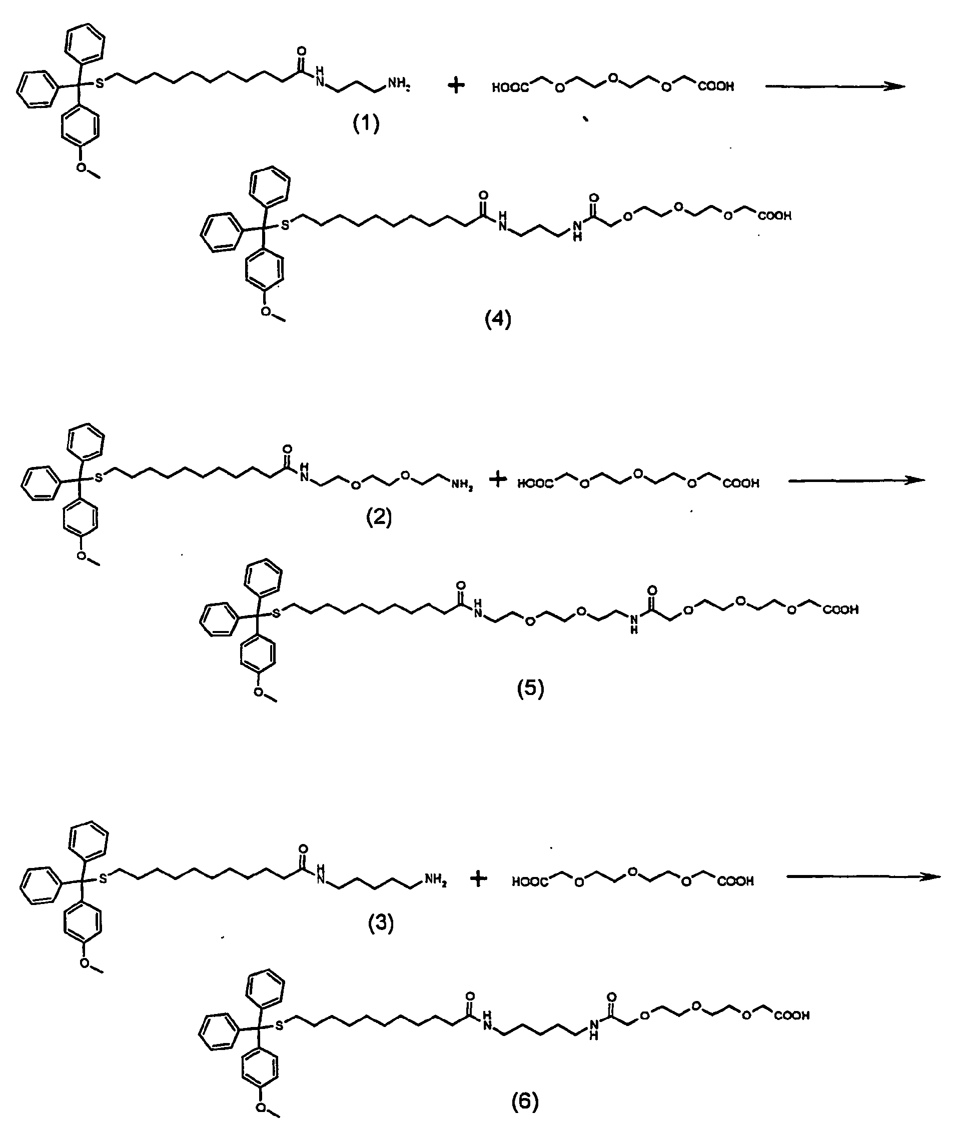

### Ansätze:

1,27 g (5,71mmol) 3,6,9-Trioxaundecandisäure
431,6mg (2,09mmol) DCC
220,2mg (1,90mmol) NHS
1,23g (1,62ml) Diisopropylethylamin (DIEA)
   a) 1,04 g (1) (1,90 mmol)
   b) 1,17 g (2) (1,90 mmol)
   c) 1,09 g (3) (1,90 mmol)

### Durchführung für a) bis c):

Jeweils 1,27 g (5,71 mmol) 3,6,9-Trioxaundecandisäure, 431,6mg (2,09mmol) DCC, 220,2mg (1,90mmol) NHS und 1,23g (1,62ml) Diisopropylethylamin (DIEA) wurden in jeweils 30ml DCM gelöst und 30 min bei Raumtemperatur gerührt. Danach wurden 1,90 mmol der Amine (1) (1,04 g), (2) (1,17 g) und (3) (1,09 g) in jeweils 20 ml DCM gelöst und unter Rühren in 10 Portionen zu den drei Reaktionsgemischen gegeben. Die Gemische wurden 3h bei Raumtemperatur gerührt, anschliessend der Harnstoff abfiltriert und das Lösungsmittel zur Trockene eingeengt. Die Produkte (4), (5) und (6) wurden in Ethylacetat gelöst und jeweils 3 mal mit 0,1 M HCl, 2 mal mit gesättigter Natriumchloridlösung und 2 mal mit destilliertem Wasser gewaschen. Anschließend wurden die Lösungen über Natriumsulfat getrocknet und zur Trockene eingeengt.

Darstellung von Anker (13) und zugehöriger Verdünner (7) und (8)

Ansätze:
Jeweils:
0,72g (0,873mmol) (5)
198 mg (0,96 mmol) DCC
101,8mg (0,873 mmol) NHS
   a) 5 ml 26%ige Ammoniaklösung
   b) 266,5 mg (263,4 µL) (4,36 mmol) 2-Aminoethanol

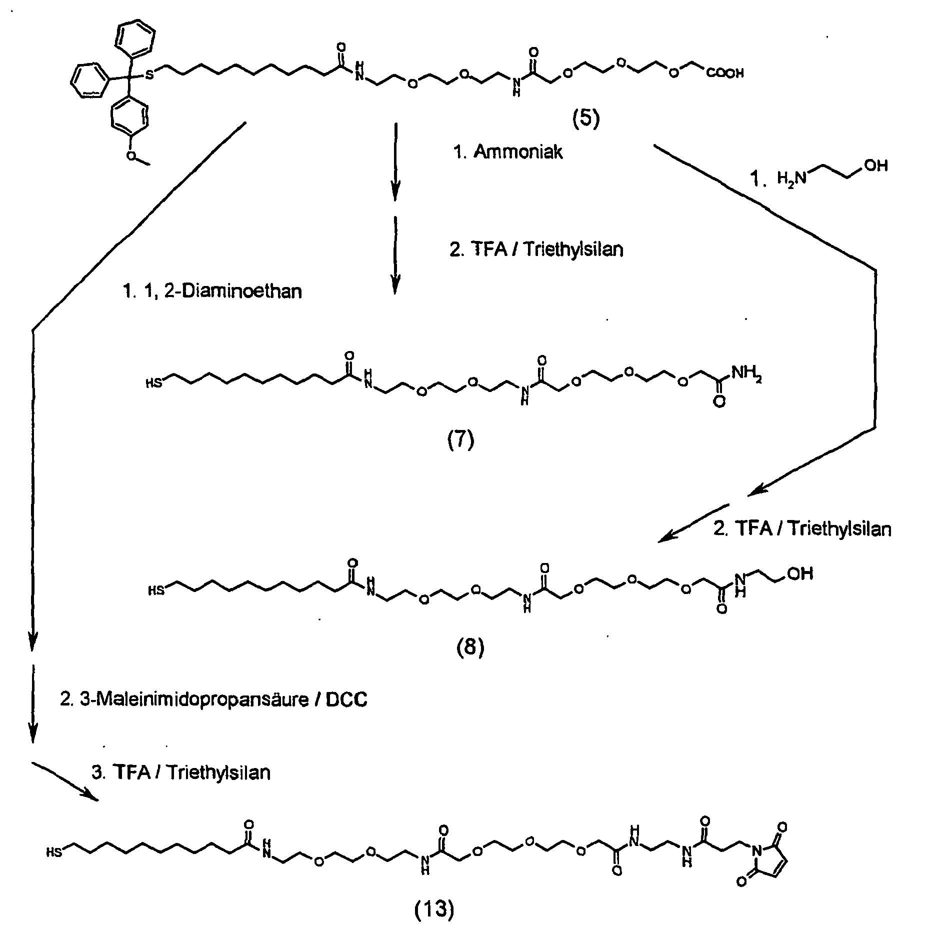

### Durchführung:

Jeweils 0,72 g (0,873 mmol) (5), 198 mg (0,96 mmol) DCC und 101,8 mg (0,873 mmol) NHS wurden in jeweils 30ml DCM gelöst und anschließend 30 min bei Raumtemperatur gerührt. Danach wurden a) 5ml 29 %ige Ammoniaklösung bzw. b) 266,5 mg (263,4 µL) (4,36 mmol) 2-Aminoethanol unter kräftigem Rühren zugegeben und weitere 2 h gerührt. Anschliessend wurde der Harnstoff abfiltriert und das Lösungsmittel zur Trockene eingeengt. Die Produkte (7) und (8) wurden in Ethylacetat (EE) gelöst und jeweils 3 mal mit 10%iger Natriumcarbonatlösung, 2 mal mit gesättigter Natriumchloridlösung und 2 mal mit destilliertem Wasser gewaschen. Die Lösungen wurden über Natriumsulfat getrocknet und zur Trockene eingeengt.

Zur Abspaltung der 4-Methoxytrityl-Schutzgruppe wurden die Verbindungen (7) und (8) in jeweils 20 ml Trifluoressigsäure gelöst, 2 ml Triethylsilan zugegeben und 30 min bei Raumtemperatur. Die Lösungen wurden zur Trockene eingeengt und die Produkte mittels präparativer HPLC gereinigt und LC/MS analysiert.
LC/MS (ber.):
(7): [M+H]⁺ 552,3 (552,7), [M+Na]⁺574,3 (574,7)
(8): [M+H]⁺ 596,3 (596,8), [M+Na]⁺ 618,3 (618,8)

200 mg (5) wurden analog 1a mit 1,2-Diaminoethan umgesetzt. Anschliessend wurde wie unter 4 beschrieben mit 3-Maleinimidopropansäure umgesetzt und die 4-Methoxytritylschutzgruppe abgespalten. Das Produkt (13) wurde mittels präparativer HPLC gereinigt und LC/MS analysiert
LC/MS (ber.): [M+H]⁺ 746,6 (746,9), [M+Na]⁺ 748,5 (748,9)

### 3) Darstellung von Ankern (11) und (12) und zugehöriger Verdünner (9) und (10)

### Ansatz:

a) 0,73g (6) (0,94 mmol)
b) 0,45g (4) (0,94 mmol)

213,3mg (1,034 mmol) DCC
108,9mg (0,940 mmol) NHS
417,9mg (411,8 µl; 2,82 mmol) 1,8-Diamino-3,6-dioxaoctan

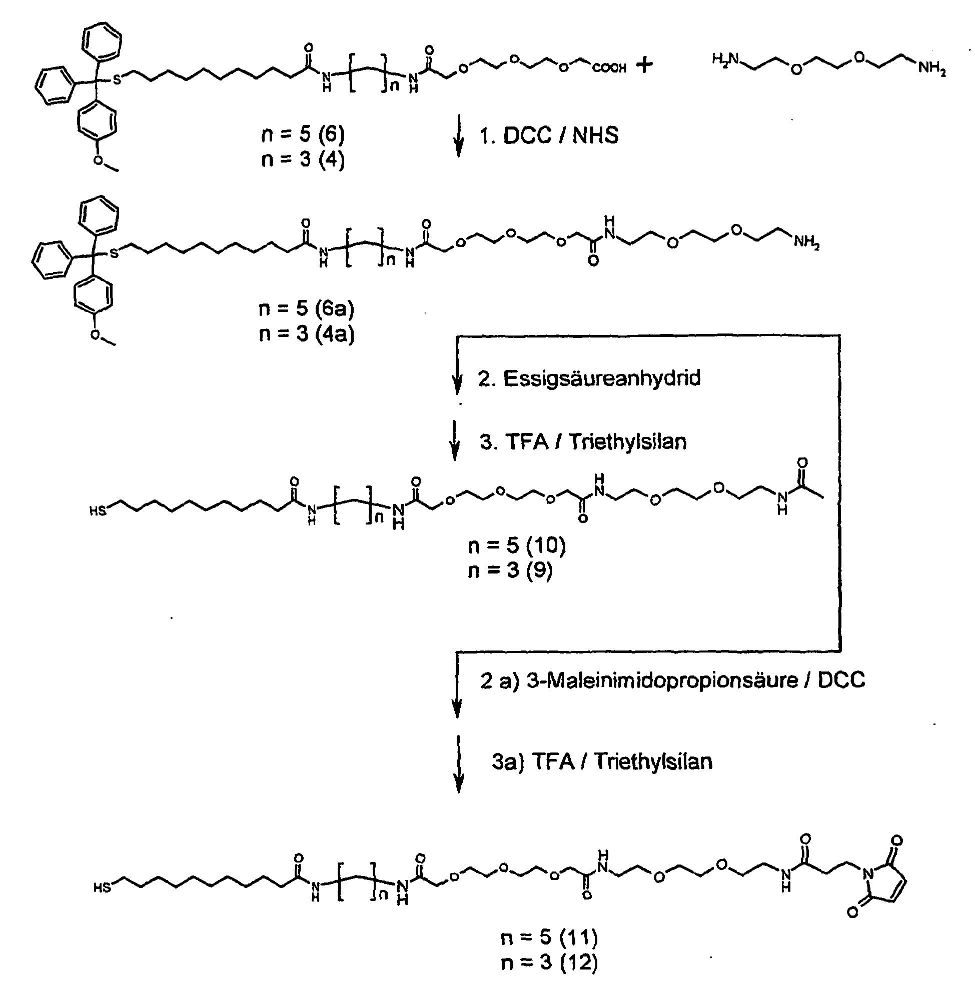

### Durchführung:

0,73g (0,94 mmol) (6) bzw. 0,45g (0,94 mmol) (4) wurden in jeweils 20ml DCM gelöst und mit jeweils 213,3mg (1,034 mmol) DCC und 108,9 mg (0,940 mmol) NHS versetzt und 30 min bei Raumtemperatur gerührt. Danach wurden jeweils 418 mg (412 µl; 2,82 mmol) 1,8-Diamino-3,6-dioxaoctan in jeweils 20 ml DCM gelöst und unter kräftigem Rühren zu den beiden Reaktionsgemischen gegeben. Nach 3 h Rühren bei Raumtemperatur wurde der Harnstoff abfiltriert und das Lösungsmittel eingeengt. Die Produkte wurden in Ethylacetat gelöst und jeweils 3 mal mit 10%iger Natriumcarbonatlösung, 2 mal mit gesättigter Natriumchloridlösung und 2 mal mit destilliertem Wasser gewaschen. Die Lösungen wurden über Natriumsulfat getrocknet und erneut zur Trockene eingengt.
Jeweils 500 mg von (4a) und (6a) wurden in jeweils 20 ml Tetrahydrofuran gelöst und mit jeweils 2 ml Essigsäureanhydrid und 2 ml Pyridin versetzt und 30 min gerührt. Anschließend wurde das Lösungsmittel eingeengt, die Produkte in Ethylacetat aufgenommen und 3 mal mit 0,1 M HCl, 2 mal mit gesättigter NaCl-Lösung und 2 mal mit destilliertem Wasser gewaschen. Die Lösungen wurden über Natriumsulfat getrocknet und zur Trockene eingeengt.

Zur Abspaltung der 4-Methoxytrityl-Schutzgrupe wurden die Produkte in jeweils 20 ml TFA und 2 ml Triethylsilan gelöst, 30 min bei Raumtemperatur gerührt und anschließend zur Trockene eingeengt. Die resultierenden Verbindungen (9) und (10) wurden mittels präparativer HPLC gereinigt und mittels LC/MS analysiert.
LC/MS (ber.):
(9) [M+H]⁺ 651,5 (651,9), [M+Na]⁺ 673,5 (673,9)
(10) [M+H]⁺ 679,6 (679,9), [M+Na]⁺701,6 (701,9)

Jeweils 200 mg von (4a) und (6a) (0,22 mmol) wurden in 10 ml DCM gelöst und jeweils eine Lösung von 43 mg (0,25 mmol) 3-Maleinimidopropansäure und 52 mg (0,25 mmol) DCC in 5 ml DCM zugegeben und 3 h bei Raumtemperatur gerührt. Anschliessend wurde der Harnstoff abfiltriert, die Lösungen zur Trockene eingeengt und zur Abspaltung der Schutzgruppe die Produkte in jeweils 10 ml TFA mit 2 ml Triethylsilan gelöst. Nach 30 min Rühren bei Raumtemperatur wurden die Lösungen zur Trockene eingeengt, die Produkte (11) und (12) mittels präparativer HPLC gereinigt und mittels LC/MS analysiert.
LC/MS (ber.):
(11) [M+H]⁺ 788,6 (788,9), [M+Na]⁺ 810,5 (810,9)
(12) [M+H]⁺ 760,5 (760,9), [M+Na]⁺782,3 (782,9)

## Patentansprüche

1. Verfahren zur Generierung einer Bindeschicht auf der Metalloberfläche eines Festphasenträgers, umfassend das Kontaktieren einer Lösung einer Vielzahl gleicher oder unterschiedlicher Ankermoleküle der Formel
HS-R-M
in der die Struktureinheit R die Ausbildung einer selbstassemblierten Monolage auf der Oberfläche ermöglicht und Festphasenträger, bestehend aus einem Substrat, das aus einem Metall gebildet wird bzw. eine Schichteines Metalls trägt, mit einer Bindeschicht, wobei die Bindeschicht erhältlich ist nach dem Verfahren eines der Ansprüche 1 bis 3
und M eine mercaptophile Kopfgruppe, bestehend aus einem Substrat, das aus einem Metall gebildet wird bzw. eine Schichteines Metalls trägt, darstellt, die mit einer Thiolgruppe unter Ausbildung einer kovalenten Bindung reagieren kann, mit der Oberfläche unter sauren Bedingungen.

2. Verfahren nach Anspruch 1, wobei zusammen mit den Ankermolekülen eine Vielzahl gleicher oder unterschiedlicher Verdünnermoleküle der allgemeinen Formel
HS-R-X
in der R wie in Anspruch 1 definiert ist und X eine nicht-mercaptophile Kopfgruppe darstellt, mit der Oberfläche in Kontakt gebracht werden.

3. Verfahren nach Anspruch 2, wobei das Verhältnis von Ankermolekülen zu Verdünnermolekülen zwischen 1:2 und 1:10.000 liegt.

4. Festphasenträger, bestehend aus einem Substrat, das aus einem Metall gebildet wird bzw. eine Schichteines Metalls trägt, mit einer Bindeschicht, wobei die Bindeschicht erhältlich ist nach dem Verfahren eines der Ansprüche 1 bis 3.

5. Festphasenträger nach Anspruch 4, wobei als Festphasenträger eine planare, nicht strukturierte Trägerplatte oder eine physikalisch strukturierte Trägerplatte eingesetzt wird, die eine Vielzahl separater Felder zur Anbindung der Anker und Verdünner aufweist.

6. Meßoberfläche, erhältlich durch kovalente Anbindung einer Vielzahl gleicher oder unterschiedlicher thioltragender oder mittels einer Thiolgruppe modifizierter biospezifischer Interaktionspartner an einen Festphasenträger nach einem der Ansprüche 4 oder 5.

7. Meßoberfläche nach Anspruch 6, wobei die gebundenen biospezifischen Interaktionspartner ausgewählt sind aus Proteinen, Peptiden, Oligonukleotiden, Kohlehydraten, Isoprenoiden, Enzymen, Lipidstrukturen, Sacchariden, Antikörpern, Peptidhormonen, Zytokinen, Antibiotika oder kleinen organischen Molekülen, wobei sich die kleinen organischen Moleküle durch ein Molekulargewicht auszeichnen das zwischen 50 und 3000 g/mol liegt.

8. Meßanordnung, umfassend eine Vielzahl von Meßoberflächen nach Anspruch 6 oder 7, wobei sich die einzelnen Meßoberflächen mindestens in der Struktur der gebundenen Interaktionspartner unterscheiden.

9. Verfahren zur Bereitstellung einer Meßanordnung nach Anspruch 8, wobei Lösungen der Interaktionspartner auf definierte, räumlich begrenzte Bereiche der Bindeoberfläche aufgebracht werden.

10. Verfahren nach Anspruch 9, wobei die Lösung der Interaktionspartner mittels einer Pipettiervorrichtung, einer Tüpfelvorrichtung, einer Mikropipettiervorrichtung oder eines Ink-Jet -Verfahrens aufgebracht wird.

11. Verfahren zur Detektion und/oder Quantifizierung einer Wechselwirkung zwischen oberflächengebundenen und freien Interaktionspartnern, umfassend das Kontaktieren der freien Interaktionspartner mit einer Meßoberfläche oder einer Meßanordnung nach einem der Ansprüche 6 bis 8.

12. Verfahren nach Anspruch 11, wobei eine Wechselwirkung der oberflächengebundenen Interaktionspartner mit einem oder mehreren Rezeptoren als freien Interaktiorispartnern stattfindet, ausgewählt aus Proteinen, DNA, RNA, Oligonukleotiden, prosthetischen Gruppen, Vitaminen, Lipiden, Mono-, Oligo- oder Polysacchariden, oder Fusionsproteinen oder synthetisierten Primern.

13. Verwendung einer Meßoberfläche oder einer Meßanordnung nach einem der Ansprüche 6 bis 8 zur Interaktionsanalyse, in Screening-Verfahren oder in der Affinitätschromatographie.

14. Verwendung einer Meßoberfläche oder einer Meßanordnung nach einem der Ansprüche 6 bis 8 in der medizinischen Diagnostik.

15. Ankermolekül der Formel
HS-R-M
wobei die Struktureinheit R die Ausbildung einer selbstassemblierten Monolage auf der Oberfläche ermöglicht und die folgende allgemeine Struktur aufweist
-(CH₂)ₐ-Q¹-(CH₂)_{b}-{[Q²-(CH₂)_{c}-[O-(CH₂)_{d}]ₑ-O-(CH₂)_{f}]_{g}-[Q³-(CH₂)_{c}-
[O-(CH₂)_{d'}]_{e'}-O-(CH₂)_{f}]_{b}}ᵢ-Q⁴-(CH₂)ⱼ-Q⁵-(CH₂)ₖ-
wobei die Variablen, jeweils unabhängig voneinander, wie folgt definiert sind und Zahlenbereiche ihre jeweiligen Grenzen sowie alle darin enthaltenen ganzen Zahlen umfassen sollen:
Q¹, Q⁵ sind -NH-C(O)-, -C(O)-NH- oder eine Bindung
Q², Q³, Q⁴ sind -NH-C(O)- oder -C(O)-NH-
a ist 5 bis 20, bevorzugt 8 bis 12, besonders bevorzugt 10;
b ist 0 bis 5, bevorzugt 0 falls Q¹ eine Bindung ist und 1 bis 10, bevorzugt 2 bis 7, besonders bevorzugt 3 bis 5 in allen anderen Fallen;
c, c' sind 1 bis 5, bevorzugt 1 bis 3, besonders bevorzugt 1;
d, d' sind 1 bis 5, bevorzugt 1 bis 3, besonders bevorzugt 2;
e, e' sind 1 bis 5, bevorzugt 1 bis 3, besonders bevorzugt 2;
f, f' sind 1 bis 5, bevorzugt 1 bis 3, besonders bevorzugt 1;
g,h sind 0 bis 3, mit der Maßgabe dass g+h ≥ 1, bevorzugt g+h = 2;
i ist 1 bis 3, bevorzugt 1 bis 2, besonders bevorzugt 1;
j ist 0 bis 5, bevorzugt 1 bis 3, besonders bevorzugt 2; und
k ist 0 bis 5
und M eine mercaptophile Kopfgruppe ausgewählt aus Iod- oder Bromacetamid, Pyridyldithioverbindungen, Michael-Akzeptoren, Acrylsäure, (Meth)Acrylsäureestern, -amiden, -lactonen, -lactamen, Methylen-gemdifluorcyclopropanen, α, β-ungesättigten Aldehyden und Ketonen sowie α, β-ungesättigten Sulfonen und Sulfonamiden darstellt, die mit einer Thiolgruppe unter Ausbildung einer kovalenten Bindung reagieren kann.

16. Ankermolekül nach Anspruch 15, wobei M die folgende allgemeine Struktur aufweist 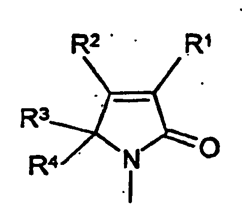 in der
R¹ und R² unabhängig voneinander Wasserstoff oder C₁-C₅ Alkyl sind,
R³ und R⁴ unabhängig voneinander Wasserstoffoder C₁-C₅ Alkyl oder R³ und R⁴ gemeinsam =O sind.

17. Verfahren zur Festphasensynthese eines Ankermoleküls nach einem der Ansprüche 15 oder 16, wobei das Ankermolekül über die Thiolgruppe an die Synthesephase gekoppelt ist und zur Bereitstellung des freien Ankermoleküls in saurem Medium abgespalten wird.

## Claims

1. Process for the generation of a binding layer on the metal surface of a solid phase carrier, comprising the step of contacting a solution of a plurality of identical or different anchor molecules represented by the formula
HS-R-M
wherein the structural moiety R provides for the formation of a self-assembling monolayer on the surface and has the following general structure
-(CH₂)ₐ-Q¹-(CH₂)_{b}-{[Q²-(CH₂)_{c}-[O-(CH₂)_{d}]ₑ-O- (CH₂)_{f}]_{g}-[Q³-(CH₂)_{c'}-
[O-(CH₂)_{d'}]_{e'}-O-(CH₂)_{f'}]ₕ}ᵢ-Q⁴-(CH₂)ⱼ-Q⁵-(CH₂)ₖ-
wherein the variables are defined independently of each other as follows and the numeric ranges comprise their respective limiting values as well as the integers contained therein:
Q¹,Q⁵ are -NH-C(O)-, -C(O)-NH- or a bond
Q², Q³, Q⁴ are -NH-C(O)- or -C(O)-NH-
a is 5 to 20, preferably 8 to 12, particularly preferred 10;
b is 0 to 5, preferably 0, if Q¹ is a bond and 1 to 10, preferably 2 to 7, particularly preferred 3 to 5 in all other cases;
c, c' are 1 to 5, preferably 1 to 3, particularly preferred 1;
d, d' are 1 to 5, preferably 1 to 3, particularly preferred 2;
e, e' are 1 to 5, preferably 1 to 3, particularly preferred 2;
f, f' are 1 to 5, preferably 1 to 3, particularly preferred 1;
g, h are 0 to 3, provided that g+h ≥ 1, preferably g+h = 2;
i is 1 to 3, preferably 1 to 2, particularly preferred 1;
j is 0 to 5, preferably 1 to 3, particularly preferred 2; and
k is 0 to 5,
and M represents a mercaptophilic head group selected from iodo- or bromoacetamide, pyridyldithio compounds, Michael acceptors, acrylic acid, (meth)acrylic acid esters, -amides, -lactones, -lactames, methylene-gem-difluorocyclopropanes, α,β-unsaturated aldehydes and ketones and α,β-unsaturated sulfones and sulfonamides, which can react with a thiol group by forming a covalent bond, with the surface under acidic conditions.

2. Process according to claim 1, wherein a plurality of identical or different diluting molecules represented by the general formula
HS-R-X
wherein R is defined as in claim 1 and X represents a non-mercaptophilic head group, is contacted with the surface together with the anchor molecules.

3. Process according to claim 2, wherein the ratio of anchor molecules to diluting molecules ranges from 1:2 to 1:10,000.

4. Solid phase carrier consisting of a substrate formed by a metal or carrying a layer of a metal, respectively, with a binding layer, wherein the binding layer is obtainable according to the process of any of claims 1 to 3.

5. Solid phase carrier according to claim 4, wherein a planar, non-structured carrier plate or a physically structured carrier plate having a plurality of separate fields for binding the anchors and diluants is used as the solid phase support.

6. Measuring surface obtainable by covalently binding a plurality of identical or different biospecific interaction partners which carry a thiol group or which are modified by means of a thiol group to a solid phase carrier according to any of claims 4 or 5.

7. Measuring surface according to claim 6', wherein the bound biospecific interaction partners are selected from proteins, peptides, oligonucleotides, carbohydrates, isoprenoids, enzymes, lipid structures, saccharides, antibodies, peptide hormones, cytokines, antibiotics or small organic molecules, the small organic molecules being **characterized by** having a molecular weight ranging from 50 to 3,000 g/mol.

8. Measuring array comprising a plurality of measuring surfaces according to claim 6 or 7, wherein the individual measuring surfaces differ from each other at least in the structure of the bound interaction partners.

9. Process for providing a measuring array according to claim 8, wherein solutions of the interaction partners are applied onto defined, spatially limited areas of the binding surface.

10. Process according to claim 9, wherein the solution of the interaction partners is applied by means of a pipetting device, a spotting device, a micropipetting device or an ink-jet process.

11. Process for the detection and/or quantification of an interaction between surface-bound and free interaction partners, comprising contacting the free interaction partners with a measuring surface or a measuring array according to any of claims 6 to 8.

12. Process according to claim 11, wherein an interaction of the surface-bound interaction partners takes place with one or more receptors as free interaction partners, selected from proteins, DNA, RNA, oligonucleotides, prosthetic groups, vitamins, lipids, mono-, oligo- or polysaccharides or fusion proteins or synthetic primers.

13. Use of a measuring surface or a measuring array according to any of claims 6 to 8 for interaction analysis, in screening processes or in affinity chromatography.

14. Use of a measuring surface or a measuring array according to any of claims 6 to 8 in medical diagnostics.

15. Anchor molecule of the formula
HS-R-M
wherein the structural moiety R provides for the formation of a self-assembling monolayer on the surface and has the following general structure
-(CH₂)ₐ-Q¹-(CH₂)_{b}-{[Q²-(CH₂)_{c}-[O-(CH₂)_{d}]ₑ-O-(CH₂)_{f}]_{g}-[Q³-(CH₂)_{c}-
[O-(CH₂)_{d'}]_{e,}-O-(CH₂)_{f'}]ₕ}ᵢ-Q⁴-(CH₂)ⱼ-Q⁵-(CH₂)ₖ-
wherein the variables are defined independently of each other as follows and the numeric ranges comprise their respective limiting values as well as the integers contained therein:
Q¹,Q⁵ are -NH-C(O)-, -C(O)-NH- or a bond
Q², Q³, Q⁴ are -NH-C(O)- or -C(O)-NH-
a is 5 to 20, preferably 8 to 12, particularly preferred 10;
b is 0 to 5, preferably 0 if Q1 is a bond and 1 to 10, preferably 2 to 7, particularly preferred 3 to 5 in all other cases;
c, c' are 1 to 5, preferably 1 to 3, particularly preferred 1;
d, d' are 1 to 5, preferably 1 to 3, particularly preferred 2;
e, e' are 1 to 5, preferably 1 to 3, particularly preferred 2;
f, f' are 1 to 5, preferably 1 to 3, particularly preferred 1;
g, h are 0 to 3, provided that g+h ≥ 1, preferably g+h = 2;
i is 1 to 3, preferably 1 to 2, particularly preferred 1;
j is 0 to 5, preferably 1 to 3, particularly preferred 2; and
k is 0 to 5,
and M represents a mercaptophilic head group selected from iodo- or bromoacetamide, pyridyldithio compounds, Michael acceptors, acrylic acid, (meth)acrylic acid esters, -amides, -lactones, -lactames, methylene-gem-difluorocyclopropanes, α,β-unsaturated aldehydes and ketones and α,β-unsaturated sulfones and sulfonamides, which can react with a thiol group by forming a covalent bond.

16. Anchor molecule according to claim 15, wherein M has the following structure 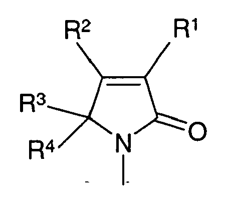 wherein
R¹ and R² are independently hydrogen or C₁-C₅ alkyl,
R³ and R⁴ are independently hydrogen or C₁-C₅ alkyl or
R³ and R⁴ together form =O.

17. Process for the solid phase synthesis of an anchor molecule according to any of claims 15 or 16, wherein the anchor molecule is coupled to the synthesis phase via the thiol group and is cleaved off in an acidic medium to provide the free anchor molecule.

## Revendications

1. Procédé de production d'une couche de liaison sur la surface métallique d'un support en phase solide, comprenant la mise en contact de la surface dans des conditions acides avec une solution d'un grand nombre de molécules d'ancrage identiques ou différentes de formule
HS-R-M,
dans laquelle l'unité structurale R permet la constitution d'une monocouche autoassemblée sur la surface, et présente la structure générale suivante :
-(CH₂)ₐ-Q¹-(CH₂)_{b}-{[Q²-(CH₂)_{c}-[O-(CH₂)_{d}]ₑ-O-(CH₂)_{f}]_{g}-[Q³-(CH₂)_{c'}-
[O-(CH₂)_{d'}]_{e'}-O-(CH₂)_{f}]ₕ}ᵢ-Q⁴-(CH₂)ⱼ-Q⁵-(CH₂)ₖ-
dans laquelle les variables, chacune indépendamment des autres, sont telles que définies ci-dessus, et les plages de valeur numérique comprennent leurs limites, ainsi que tous les nombres entiers situés entre ces dernières :
Q¹, Q⁵ sont -NH-C(O)-, -C(O)-NH- ou une liaison,
Q², Q³, Q⁴ sont -NH-C(O)- ou -C(O)-NH-
a vaut 5 à 20, de préférence 8 à 12, d'une manière particulièrement préférée 10 ;
b vaut 0 à 5, de préférence 0 si Q¹ est une liaison et 1 à 10, de préférence 2 à 7, d'une manière particulièrement préférée 3 à 5 dans tous les autres cas ;
c, c' valent 1 à 5, de préférence 1 à 3, d'une manière particulièrement préférée 1 ;
d, d' valent 1 à 5, de préférence 1 à 3, d'une manière particulièrement préférée 2 ;
e, e' valent 1 à 5, de préférence 1 à 3, d'une manière particulièrement préférée 2 ;
f, f valent 1 à 5, de préférence 1 à 3, d'une manière particulièrement préférée 1 ;
g, h valent 0 à 3, avec la condition que g+h≥1, de préférence g+h = 2 ;
i vaut 1 à 3, de préférence 1 à 2, d'une manière particulièrement préférée 1 ;
j vaut 0 à 5, de préférence 1 à 3, d'une manière particulièrement préférée 2 ; et
k vaut 0 à 5, et
M est un groupe de tête mercaptophile, choisi parmi l'iodo- ou le bromoacétamide, les composés pyridyldithio, les accepteurs de Michael, l'acide acrylique, les esters, amides, lactones et lactames de l'acide (méth)acrylique, les méthylène-gem-difluorocyclopropanes, les aldéhydes et cétones α,β-insaturés, ainsi que les sulfones et les sulfonamides α,β-insaturés, groupe de tête qui peut réagir avec un groupe thiol avec formation d'une liaison covalente.

2. Procédé selon la revendication 1, dans lequel on met en contact avec la surface, en même temps que les molécules d'ancrage, un grand nombre de molécules de dilution identiques ou différentes de formule générale
HS-R-X
dans laquelle R est tel que défini dans la revendication 1, et X est un groupe de tête non mercaptophile.

3. Procédé selon la revendication 2, dans lequel le rapport des molécules d'ancrage aux molécules de dilution est compris entre 1:2 et 1:10 000.

4. Support en phase solide étant constitué d'un substrat formé à partir d'un métal ou portant une couche métallique, et une couche de liaison, la couche de liaison étant susceptible d'être obtenue par le procédé selon l'une des revendications 1 à 3.

5. Support en phase solide selon la revendication 4, le support en phase solide utilisé étant une plaque support plane, non structurée, ou une plaque support physiquement structurée, qui comprend un grand nombre de champs distincts pour liaison des ancres et des diluants.

6. Surface de mesure susceptible d'être obtenue par liaison covalente d'un grand nombre de partenaires d'interaction biospécifiques, identiques ou différents, portant un groupe thiol ou modifiés par un groupe thiol, à un support en phase solide selon l'une des revendications 4 ou 5.

7. Surface de mesure selon la revendication 6, dans laquelle les partenaires d'interaction biospécifiques liés sont choisis parmi les protéines, les peptides, les oligonucléotides, les hydrates de carbone, les isoprénoïdes, les enzymes, les structures lipidiques, les saccharides, les anticorps, les hormones peptidiques, les cytokines, les antibiotiques ou les petites molécules organiques, les petites molécules organiques étant **caractérisées par** une masse moléculaire comprise entre 50 et 3 000 g/mol.

8. Système de mesure comprenant un grand nombre de surfaces de mesure selon la revendication 6 ou 7, les surfaces de mesure individuelles se distinguant au moins par la structure des partenaires d'interaction liés.

9. Procédé pour mettre à disposition un système de mesure selon la revendication 8, dans lequel des solutions des partenaires d'interaction sont appliquées sur des zones définies et spatialement délimitées de la surface de liaison.

10. Procédé selon la revendication 9, dans lequel la solution des partenaires d'interaction est appliquée à l'aide d'un dispositif de pipettage, d'un dispositif de tamponnage, d'un dispositif de micro-pipettage ou par un procédé au jet d'encre.

11. Procédé pour la détection et/ou la quantification d'une interaction entre des partenaires d'interaction liés à une surface et des partenaires d'interaction libres, comprenant la mise en contact des partenaires d'interaction libres avec une surface de mesure ou un système de mesure selon l'une des revendications 6 à 8.

12. Procédé selon la revendication 11, dans lequel a lieu une interaction des partenaires d'interaction liés à la surface avec un ou plusieurs récepteurs jouant le rôle de partenaires d'interaction libres, choisis parmi les protéines, l'ADN, l'ARN, les oligonucléotides, les groupes prosthétiques, les vitamines, les lipides, les mono-, oligo-ou polysaccharides, ou les protéines de fusion ou les amorces synthétisées.

13. Utilisation d'une surface de mesure ou d'un système de mesure selon l'une des revendications 6 à 8 pour l'analyse des interactions, dans le procédé de criblage pour chromatographie d'affinité.

14. Utilisation d'une surface de mesure ou d'un système de mesure selon l'une des revendications 6 à 8 en diagnostic médical.

15. Molécule d'ancrage de formule
HS-R-M,
dans laquelle l'unité structurale R autorise la constitution d'une couche autoassemblée sur la surface, et présente la structure générale suivante :
-(CH₂)ₐ-Q¹-(CH₂)_{b}-{[Q²-(CH₂)_{c}-[O-(CH₂)_{d}]ₑ-O-(CH₂)_{f}]_{g}-[Q³-(CH₂)_{c'}-
[O-(CH₂)_{d'}]_{e'}-O-(CH₂)_{f'}]ₕ}ᵢ-Q⁴-(CH₂)ⱼ-Q⁵-(CH₂)ₖ-
dans laquelle les variables, chacune indépendamment des autres, sont telles que définies ci-dessus, et les plages de valeur numérique comprennent leurs limites, ainsi que tous les nombres entiers situés entre ces dernières :
Q¹, Q⁵ sont -NH-C(O)-, -C(O)-NH- ou une liaison,
Q², Q³, Q⁴ sont -NH-C(O)- ou -C(O)-NH-
a vaut 5 à 20, de préférence 8 à 12, d'une manière particulièrement préférée 10 ;
b vaut 0 à 5, de préférence 0 si Q¹ est une liaison et 1 à 10, de préférence 2 à 7, d'une manière particulièrement préférée 3 à 5 dans tous les autres cas ;
c, c' valent 1 à 5, de préférence 1 à 3, d'une manière particulièrement préférée 1 ;
d, d' valent 1 à 5, de préférence 1 à 3, d'une manière particulièrement préférée 2 ;
e, e' valent 1 à 5, de préférence 1 à 3, d'une manière particulièrement préférée 2 ;
f, f valent 1 à 5, de préférence 1 à 3, d'une manière particulièrement préférée 1 ;
g, h valent 0 à 3, avec la condition que g+h≥1, de préférence g+h = 2 ;
i vaut 1 à 3, de préférence 1 à 2, d'une manière particulièrement préférée 1 ;
j vaut 0 à 5, de préférence 1 à 3, d'une manière particulièrement préférée 2 ; et
k vaut 0 à 5, et
M est un groupe de tête mercaptophile, choisi parmi l'iodo- ou le bromoacétamides, les composés pyridyldithio, les accepteurs de Michael, l'acide acrylique, les esters, amides, lactones et lactames de l'acide (méth)acrylique, les méthylène-gem-difluorocyclopropanes, les aldéhydes et cétones α,β-insaturés, ainsi que les sulfones et les sulfonamides α,β-insaturés, groupe de tête qui peut réagir avec un groupe thiol avec formation d'une liaison covalente.

16. Molécule d'ancrage selon la revendication 15, M ayant la structure générale suivante 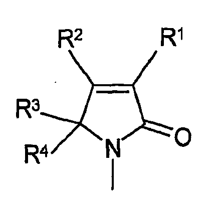 dans laquelle
R¹ et R² représentent chacun indépendamment de l'autre un atome d'hydrogène ou un radical alkyle en C₁ à C₅,
R³ et R⁴ représentent chacun indépendamment de l'autre un atome d'hydrogène ou un radical alkyle en C₁ à C₅, ou encore R³ et R⁴ forment ensemble =O.

17. Procédé de synthèse en phase solide d'une molécule d'ancrage selon l'une des revendications 15 ou 16, dans lequel la molécule d'ancrage est couplée à la phase de synthèse par l'intermédiaire du groupe thiol, et est libérée en milieu acide, pour mettre à disposition la molécule d'ancrage libre.
